# EUROPEAN PATENT APPLICATION

(11) **EP 1 156 056 A1**
(43) Date of publication of application: **21.11.2001**
(21) Application number: 00901984.5
(22) Date of filing: 28.01.2000
(51) Int. Cl.: C07H 17/02, A61K 31/706, A61P 35/00

(54) **5-GLYCOSYLOXY-6-HYDROXYNAPHTHO 2,3-f]QUINOLINE-7,12-DIONE HAVING ANTICANCER ACTIVITIES**

(30) Priority: 29.01.1999 JP 2332199
(71) Applicant: ZAIDAN HOJIN BISEIBUTSU KAGAKU KENKYU KAI, Shinagawa-ku, Tokyo 141-0021 (JP)
(72) Inventor: TAKEUCHI, Tomio, New Fuji Manshion 701, Tokyo 141-0022 (JP); UMEZAWA, Sumio, Tokyo 160-0022 (JP); TSUCHIYA, Tsutomu, Yokohama-shi, Kanagawa 224-0006 (JP); TAKAGI, Yasushi, Yokohama-shi, Kanagawa 224-0805 (JP); SOHTOME, Hiromi, Kawasaki-shi, Kanagawa 213-0023 (JP)
(74) Representative: Bratel, Gérard
(86) International application number: JP0000479
(87) International publication number: WO0044762

(57) **Abstract**

As novel compounds which exhibit reduced toxicities but enhanced anticancer activities in comparison with those of known carcinostatic anthracycline derivatives, there is provided a compound of general formula (I) 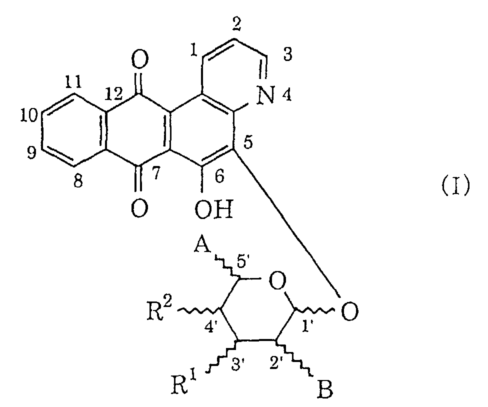 wherein A is methyl group or CF₃ group, and B is hydrogen atom, or an electron-withdrawing group chosen from halogeno group and others, provided that B stands for hydrogen atom when A is CF₃ and that B stands for an electron-withdrawing group when A is methyl group, and R¹ and R² each are hydroxyl group, amino group or an acyloxy group or an aminoalkanoyloxy group, and others, or a pharmaceutically acceptable salt thereof. The compound of general formula (I) possesses activities inhibitory to proliferation of various human cancer cells and is of lower toxicity than that of adriamycin.

Further, there is provided, as novel and water-soluble carcinostatic compounds, a compound of general formula (V) 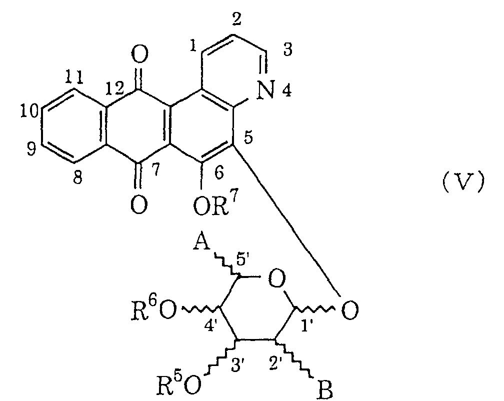 wherein A and B have the same meanings as defined above, and R⁵ is hydrogen atom or an ω-amino acid residue, and R⁶ and R⁷ have the same meanings as defined for R⁵, provided that R⁵, R⁶ and R⁷ do not represent hydrogen atom, simultaneously.

## Description

### TECHNICAL FIELD

This invention relates to a 5-glycosyloxy-6-hydroxynaphtho[2,3-f]quinoline-7,12-dione as novel compound having anticancer or antitumor activities. This invention further relates to an anticancer or antitumor composition comprising as an active ingredient a 5-glycosyloxy-6-hydroxynaphtho[2,3-f]quinoline-7,12-dione.

### BACKGROUND ART

As the antibiotics of the anthracycline type are known daunomycin(named also as daunorubicin) and adriamycin (named also as doxorubicin), and they have broad anticancer spectra against experimental tumors. In particular, adriamycin has been utilized widely in clinical applications as a chemotherapeutic anticancer agent, but the toxicity thereof to mammals must be said to be considerably high.

On the other hand, there exists 5,6-dihydroxynaphtho [2,3-f]quinoline-7,12-dione represented by the following formula (A) 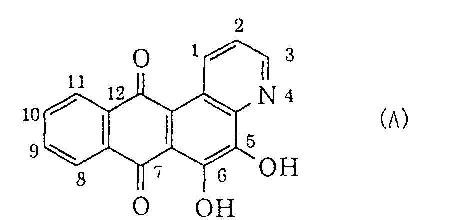 which is a blue dye known under the name of Alizarin Blue. The compound of formula (A) above and any derivatives thereof have never been used as a medicine, particularly as antitumor or anticancer agent, so far as we, the inventors of this invention, know.

Further, there have hitherto been utilized many different types of chemical compounds in chemotherapeutic treatments of cancers. As one class of such chemical compounds, there are mentioned anthracycline antibiotics as important anticancer agents. We, the inventors of this invention, have already synthesized various types of novel anthracycline derivatives having a fluoro-sugar moiety and have made it clear that many of said novel anthracycline derivatives exhibit remarkable antitumor activities and have relatively low toxicities (refer, for example, to Japanese Patent Application Kokai Hei 9-132589, United States Patent Specification No.5,789,386 and European Patent Application First Publication No. EP00761678A1). It has been recognized that said anthracycline derivatives having the fluoro-sugar moiety as synthesized by the present inventors have higher antitumor activities but lower toxicities against mammal, including human, as compared with those of adriamycin. However, the toxicities of said anthracycline derivatives having a fluoro-sugar moiety against human have yet not been recognized to be lowered enough and satisfactorily. Thus, in view of the level of toxicities of said anthracycline derivatives, it was foreseeable that patients who received administration of said anthracycline derivatives get some trouble or injury during the therapy as made therewith.

### DISCLOSURE OF THE INVENTION

Accordingly, there exist keen demands in the art to provide such novel compounds which exhibit remarkably reduced toxicities than those of the already known anthracycline derivatives and which have anticancer activities.

Under the circumstances, we have carried out our investigations with our intention of synthesizing such novel compounds which are quite different in the skeletal chemical structure from the known anthracyclines and which possess anticancer activities. As a part of these investigations, we have succeeded in synthesizing a variety of novel glycoside compounds in which various kinds of glycosyl groups are bound to the 5-hydroxyl group of the Alizarin Blue compound of formula (A) above and in which the Alizarin Blue molecule is contained as aglycone. Further, we have found that a class of glycoside compounds, which are represented collectively by the general formula (I) shown below, possesses strong carcinostatic activities against a variety of cancer cells and shows remarkably reduced toxicities, as compared with adriamycin. Based on these findings, this invention has been completed.

According to a first aspect of this invention, therefore, there is provided a 5-L or D-glycosyloxy-6-hydroxynaphtho [2,3-f]quinoline-7,12-dione represented by the following general formula (I) 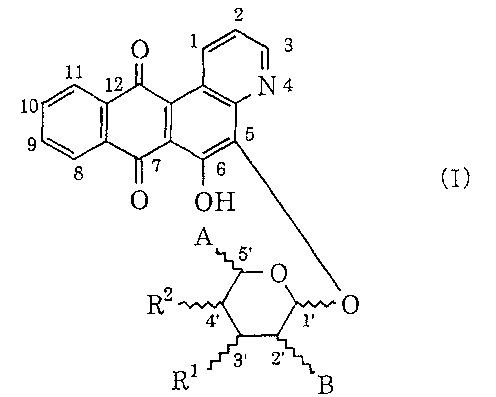 wherein A is methyl group or trifluoromethyl group, B is a hydrogen atom or an electron-withdrawing group chosen from fluoro group, chloro group, bromo group, iodo group, difluoro group (-F₂), an alkoxy group of 1-5 carbon atoms and cyano group (-CN), with provisos that when A is trifluoromethyl group, B stands for hydrogen atom, and that when A is methyl group, B stands for such electron-withdrawing group as above, and R¹ is either hydroxyl group or amino group, or a hydroxyl group having been esterified with an α-amino acid residue represented by the following formula (a) 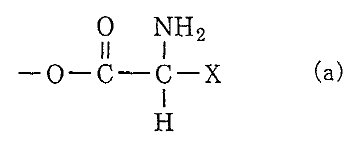 where X is hydrogen atom or such an alkyl group or such a substituted alkyl group which is known to be combined to the α-carbon atom of a known α-amino acid molecule; or R¹ is a hydroxyl group having been esterified with an acyl group represented by the following formula (b) 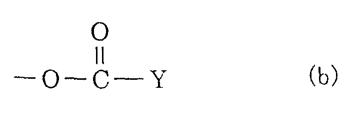 where Y is hydrogen atom or a lower alkyl group, or Y is an aryl group, particularly phenyl group or a substituted phenyl group, or Y is an aralkyl group, particularly benzyl group or a substituted benzyl group; or R¹ is a hydroxyl group having been O-glycosylated with a pentose or a hexose, and R² has the same meaning as defined for R¹, and a pharmaceutically acceptable acid-addition salt thereof.

The compound of general formula (I) according to the first aspect of this invention can form an acid-addition salt with a pharmaceutically acceptable acid at the amino group or imino group of the compound. Pharmaceutically acceptable inorganic acids usable for this purpose are, for example, hydrochloric acid, sulfuric acid, nitric acid or phosphoric acid, and pharmaceutically acceptable organic acids are, for example, acetic acid, trifluoroacetic acid, propionic acid, malonic acid, lactic acid, tartaric acid or methanesulfonic acid.

The glycosyl group which is present in the compound of general formula (I) according to the first aspect of this invention and which is represented by the following formula (c) 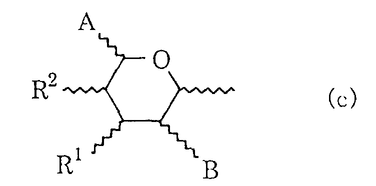 may be such an L- or D-glycosyl group which has the configuration of 2,6-dideoxy- or 6-deoxy-α or β-L or D-talopyranosyl group; or 2,6-dideoxy or 6-deoxy-α or β-L or D-galactopyranosyl group; or 2,6-dideoxy or 6-deoxy-α or β-L or D-mannopyranosyl group; or 2,6-dideoxy-α or β-L or D-lyxo-hexopyranosyl group.

[A] The compound of general formula (I) according to the first aspect of this invention includes a 5-(2,6-dideoxy or 6-deoxy-2-substituted-3-O-substituted or unsubstituted-or 4-O-substituted or unsubstituted- or 3,4-O-di-substituted or unsubstituted-L- or D-glycosyloxy)-6-hydroxynaphtho [2,3-f]quinoline-7,12-dione represented by the following general formula (Ia) 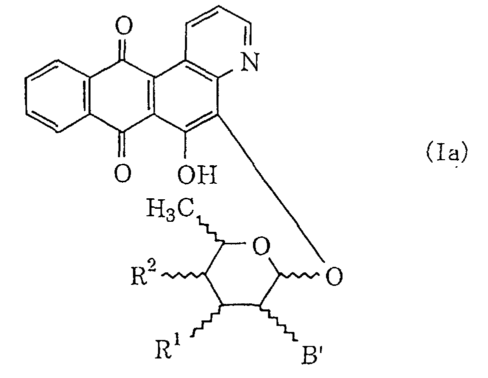 wherein B' is an electron-withdrawing group chosen from fluoro group, chloro group, bromo group, iodo group, difluoro group (-F₂), an alkoxy group of 1-5 carbon atoms and cyano group (-CN), and R¹ is either hydroxyl group or amino group, or a hydroxyl group having been esterified with an α-amino acid residue of the following formula (a) 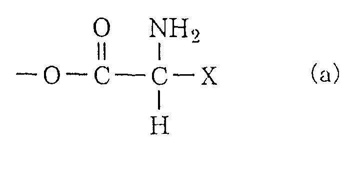 where X is hydrogen atom or such an alkyl group or such a substituted alkyl group which is known to be combined to the α-carbon atom of a known α-amino acid molecule; or R¹ is a hydroxyl group having been esterified with an acyl group of the following formula (b) 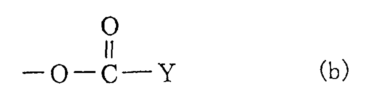 where Y is a lower alkyl group, or an aryl group, particularly phenyl group or a substituted phenyl group, or Y is an aralkyl group, particularly benzyl group or a substituted benzyl group; or R¹ is a hydroxyl group having been O-glycosylated with a pentose or a hexose, and R² has the same meaning as defined for R¹.

The glycosyl group, which is present in the compound of general formula (Ia) above-mentioned and which is represented by the following formula (c') 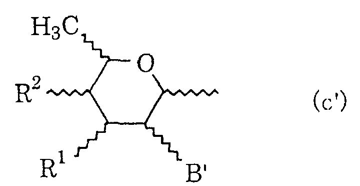 wherein B', R¹ and R² have the same meanings as defined above may be such a glycosyl group which has the configuration of 2,6-dideoxy or 6-deoxy-α or β-L or D-talopyranosyl group; or 2,6-dideoxy or 6-deoxy-α or β-L or D-galactopyranosyl group; or 2, 6-dideoxy or 6-deoxy-α or β-L or D-mannopyranosyl group.

The glycosyl group of formula (c') above may be a 4-amino-2,4,6-trideoxy-2-substituted-talopyranosyl or galactopyranosyl or mannopyranosyl group; or a 3-amino-2, 3, 6-trideoxy-2-substituted-talopyranosyl or galactopyranosyl or mannopyranosyl group, in particular.

The compound of general formula (Ia) above may be such compound of general formula (Ia) where B' is an electron-withdrawing group chosen from fluoro group, chloro group, bromo group, difluoro group, methoxy group, ethoxy group and cyano group, and R¹ is hydroxyl group, amino group, glycyloxy group, alanyloxy group or acetoxy group, and R² is hydroxyl group, amino group, glycyloxy group, alanyloxy group or acetoxy group.

The compound of general formula (Ia) above includes a 5-(2, 6-dideoxy or 6-deoxy-2-substituted-L or D-talopyranosyloxy or galactopyranosyloxy or mannopyranosyloxy)-6-hydroxynaphtho [2,3-f]quinoline-7,12-dione represented by the following general formula (Ia¹) 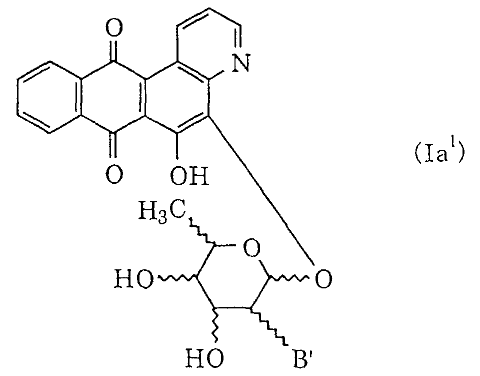 wherein B' is an electron-withdrawing group having the same meaning as defined above.

As examples of the compound of general formula (Ia¹) above, there are given 5-(2,6-dideoxy-2-fluoro-α-L-talopyranosyloxy)-6-hydroxynaphtho[2,3-f]quinoline-7,12-dione (hereinafter referred sometimes to as Compound A of this invention) represented by the following formula (Ia¹-1) 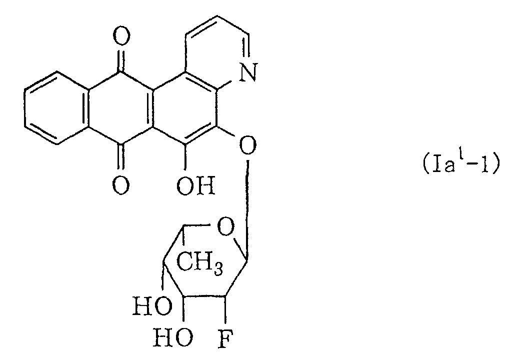 and 5-(6-deoxy-2-O-methyl-a-L-talopyranosyloxy)-6-hydroxynaphtho[2,3-f]quinoline-7,12-dione (hereinafter referred sometimes to as Compound B of this invention) represented by the following formula (Ia¹-2) 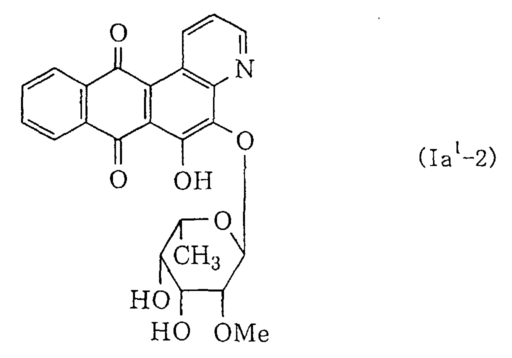 wherein Me is methyl group.

As a further example of the compound of general formula (Ia¹) above, there is given 5-(2,6-dideoxy-2-fluoro-α-L-mannopyranosyloxy)-6-hydroxynaphtho[2,3-f]quinoline-7,12-dione (hereinafter referred sometimes to as Compound C of this invention) represented by the following formula (Ia¹-3)

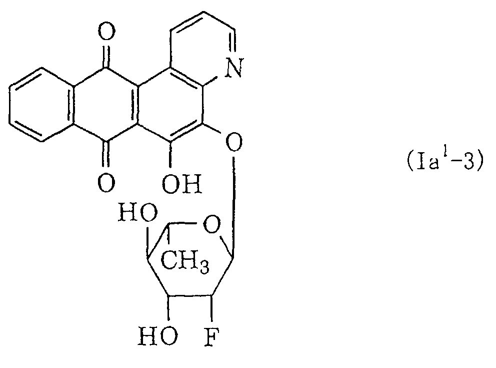

As further examples of the compound of general formula (Ia¹) above, there are given 5-(2,6-dideoxy-2-fluoro-α-L-galactopyranosyloxy)-6-hydroxynaphtho[2,3-f]quinoline-7, 12-dione(hereinafter referred sometimes to as Compound D of this invention) represented by the following formula (Ia¹-4) 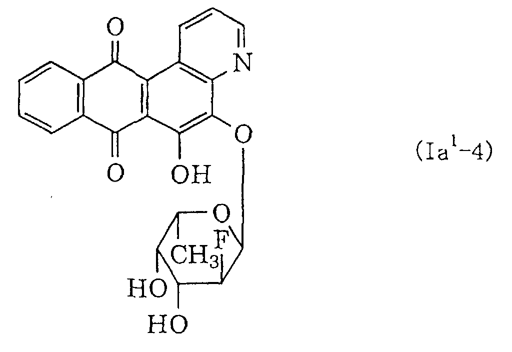 and 5-(2,6-dideoxy-2-fluoro-β-L-galactopyranosyloxy)-6-hydroxynaphtho[2,3-f]quinoline-7,12-dione (hereinafter referred sometimes to as Compound E of this invention) represented by the following formula (Ia¹-5)

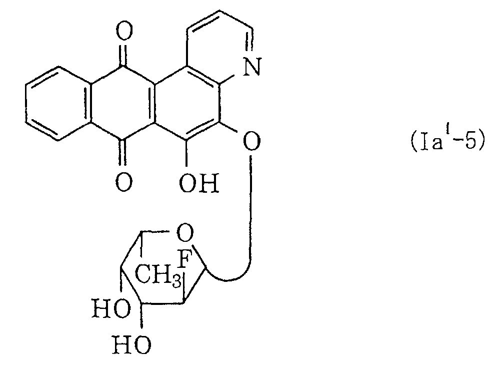

Further, the compound of general formula (Ia) above may be a 5-(4-amino-2,4,6-trideoxy-2-substituted-L or D-talopyranosyloxy or galactopyranosyloxy or mannopyranosyloxy) -6-hydroxynaphtho[2,3-f]quinoline-7,12-dione represented by the following formula (Ia²) 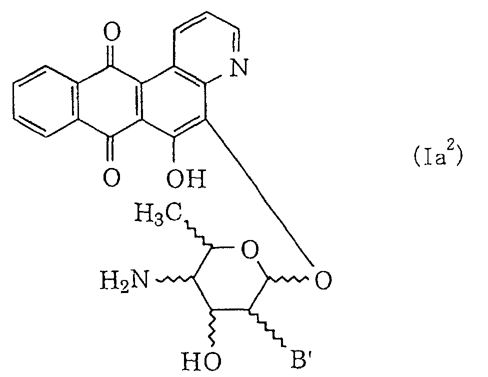 wherein B' is an electron-withdrawing group having the same meaning as defined above.

As examples of the compound of general formula (Ia²) above, there are given 5-(4-amino-2,4,6-trideoxy-2-fluoro-α-L-mannopyranosyloxy)-6-hydroxynaphtho[2,3-f]quinoline-7,12-dione (hereinafter referred sometimes to as Compound F of this invention) represented by the following formula (Ia²-1) 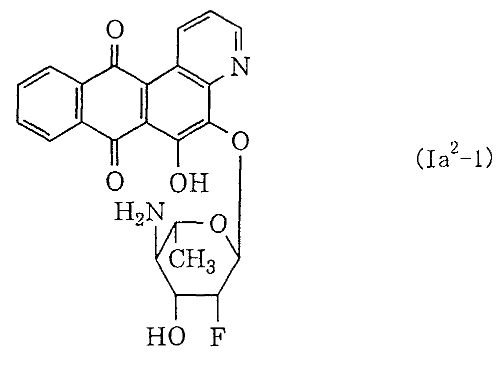 and 5-(4-amino-2,4,6-trideoxy-2-fluoro-α-L-talopyranosyloxy) -6-hydroxynaphtho[2,3-f]quinoline-7,12-dione (hereinafter sometimes referred to as Compound G of this invention) represented by the following formula (Ia²-2)

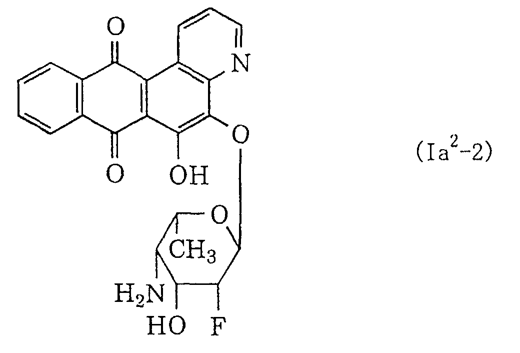

Further, the compound of general formula (Ia) above may be a 5-(3-amino-2,3,6-trideoxy-2-substituted-L or D-talopyranosyloxy or galactopyranosyloxy or mannopyranosyloxy) -6-hydroxynaphtho[2,3-f]quinoline-7,12-dione represented by the following general formula (Ia³) 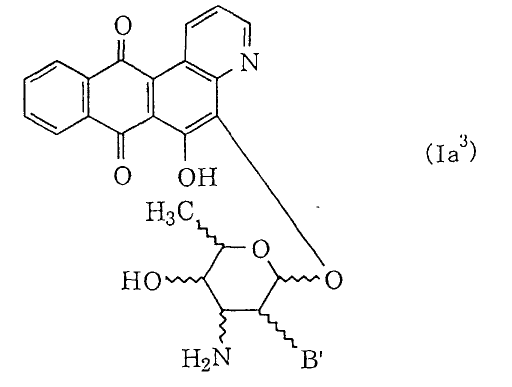 wherein B' is an electron-withdrawing group having the same meaning as defined above.

One example of the compound of general formula (Ia³) above is 5-(3-amino-2,3,6-trideoxy-2-fluoro-α-L-talopyranosyloxy) -6-hydroxynaphtho[2,3-f]quinoline-7,12-dione (hereinafter sometimes referred to as Compound H of this invention) represented by the following formula (Ia³-1)

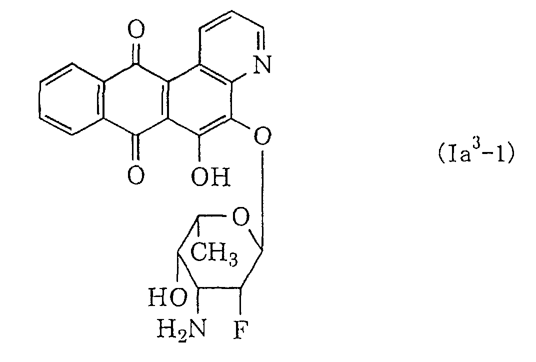

Further, the compound of general formula (Ia) above may be a 5-(2,6-dideoxy-2-substituted-3-mono-O-aminoalkanoyl-or 4-mono-O-aminoalkanoyl- or 3,4-di-O-aminoalkanoyl-L or D-talopyranosyloxy or galactopyranosyloxy or mannopyranosyloxy) -6-hydroxynaphtho[2,3-f]quinoline 7,12-dione represented by the following general formula (Ia⁴) 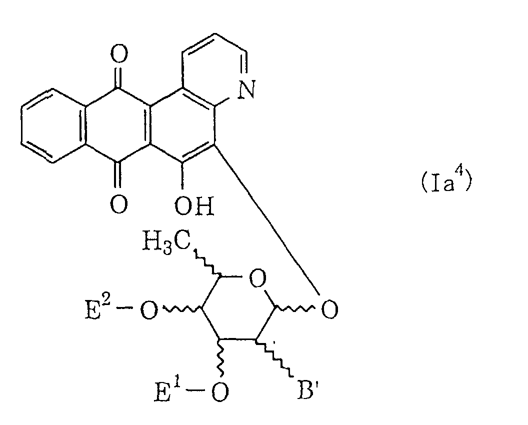 wherein B' is an electron-withdrawing group having the same meaning as defined above, and E¹ is hydrogen atom or an α-amino acid residue which is glycyl group, alanyl group, valyl group, leucyl group, isoleucyl group or phenylalanyl group, and E² is hydrogen atom or an α-amino acid residue which is glycyl group, alanyl group, valyl group, leucyl group, isoleucyl group or phenylalanyl group, provided that E¹ and E² do not represent hydrogen atom, simultaneously.

As examples of compound of general formula (Ia⁴) above, there are given 5-(3-O-L-alanyl-2,6-dideoxy-2-fluoro-α-L-talopyranosyloxy)-6-hydroxynaphtho[2,3-f]quinoline-7,12-dione (hereinafter sometimes referred to as Compound I of this invention) represented by the following formula (Ia⁴-1) 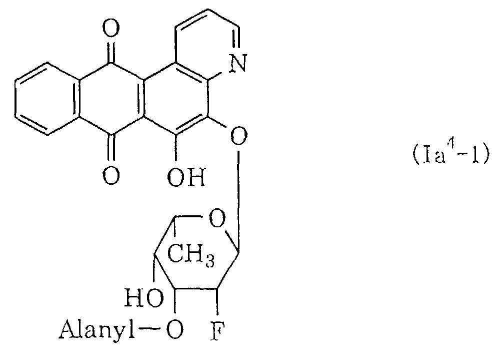 wherein Alanyl stands for L-alanyl group of the formula H₃CCH(-NH₂)-CO-; and 5- (4-O-L-alanyl-2, 6-dideoxy-2-fluoro-a-L-talopyranosyloxy)-6-hydroxynaphtho[2,3-f]quinoline-7, 12-dione (hereinafter sometimes referred to as Compound J of this invention) represented by the following formula (Ia⁴-2) 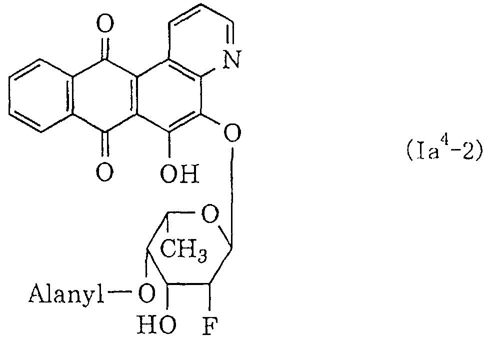 wherein Alanyl has the same meaning as defined above; and 5- (3,4 -di-O-L-alanyl-2,6-dideoxy-2-fluoro-α-L-talopyranosyloxy)-6-hydroxynaphtho[2,3-f]quinoline-7,12-dione (hereinafter sometimes referred to as Compound K of this invention) represented by the following formula (Ia⁴-3) 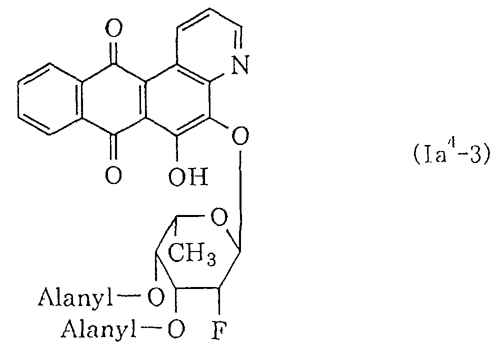 wherein Alanyl has the same meaning as defined above.

The compound of general formula (Ia), which is included within the compounds of general formula (I) according to the first aspect of this invention, may also be a 5-(2,6-dideoxy-2-substituted-3-mono-O-acyl or 4-mono-O-acyl or 3,4-di-O-acyl-L or D-talopyranosyloxy or galactopyranosyl or mannopyranosyloxy)-6-hydroxynaphtho[2,3-f]quinoline-7,12-dione represented by the following general formula (Ia⁵) 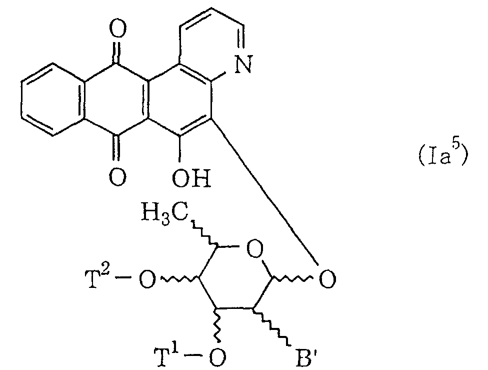 wherein B' is an electron-withdrawing group having the same meaning as defined above, and T¹ is hydrogen atom or an acyl group which is formyl group, acetyl group, propionyl group, benzoyl group or benzylcarbonyl group, and T² has the same meaning as defined above for T¹, provided that T¹ and T² do not represent hydrogen atoms, simultaneously.

[B] The compound of general formula (I) according to the first aspect of this invention may further include a 5-(2,6-dideoxy-6,6,6-trifluoro-3-O-substituted or unsubstituted- or 4-O-substituted or unsubstituted- or 3,4-O-di-substituted or unsubstituted-glycosyloxy)-6-hydroxynaphtho[2,3-f]quinoline-7,12-dione represented by the following formula (Ib) 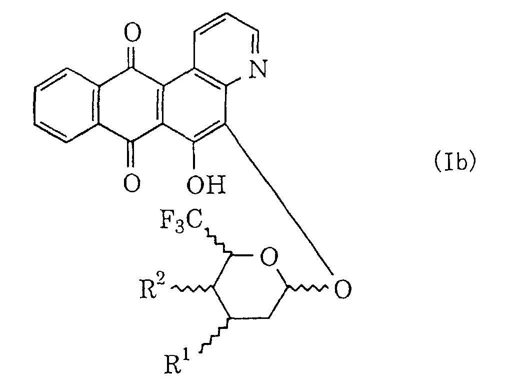 wherein R¹ is hydroxyl group or amino group, or a hydroxyl group having been esterified with an α-amino acid residue of the following formula(a) 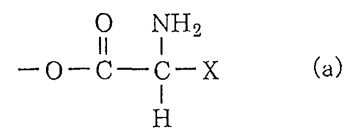 where X stands for hydrogen atom or such an alkyl group or such a substituted alkyl group which is known to be combined to the α-carbon atom of a known α-amino acid molecule; or R¹ is a hydroxyl group having been esterified with an acyl group of the following formula (b) 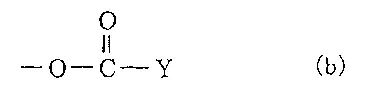 where Y stands for hydrogen atom or a lower alkyl group, or an aryl group, particularly phenyl group or a substituted phenyl group, or Y is an aralkyl group, particularly benzyl group or a substituted benzyl group; or R¹ is a hydroxyl group having been O-glycosylated with a pentose or a hexose, and R² has the same meaning as defined for R¹.

The glycosyl group existing in the compound of general formula (Ib) above and being represented by the following formula (c") 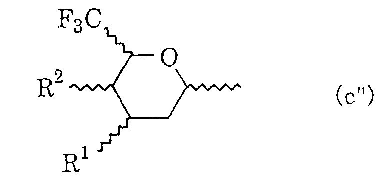 may also be such a glycosyl group which has the configuration of 2,6-dideoxy-6,6,6-trifluoro-α or β-L or D-ribo-hexopyranosyl group; or 2, 6-dideoxy-6, 6, 6-trifluoro-α or β-L or D-arabino-hexopyranosyl group; or 2,6-dideoxy-6,6,6-trifluoro-α or β-L or D-xylo-hexopyranosyl group; or 2,6-dideoxy-6,6,6-trifluoro -α or β-L or D-lyxo-hexopyranosyl group.

The compound of general formula (Ib) above may be such a compound of said formula (Ib) wherein R¹ is hydroxyl group, amino group, glycyloxy group, alanyloxy group or acetoxy group, and R² is hydroxyl group, amino group, glycyloxy group, alanyloxy group or acetoxy group.

The compound of general formula (Ib) above may be a 5-(2,6-dideoxy-6,6,6-trifluoro-4-substituted or unsubstituted -α or β-L or D-ribo-hexopyranosyloxy or 2,6-dideoxy-6,6,6-trifluoro-4-substituted or unsubstituted-α or β-L or D-arabino-hexopyranosyloxy or 2,6-dideoxy-6,6,6-trifluoro-4-substituted or unsubstituted-α or β-L or D-xylo-hexopyranosyloxy or 2,6-dideoxy-6, 6, 6-trifluoro-4-substituted or unsubstituted-α or β-L or D-lyxo-hexopyranosyloxy)-6-hydroxynaphtho[2,3-f]quinoline-7,12-dione represented by the following general formula (Ib¹) 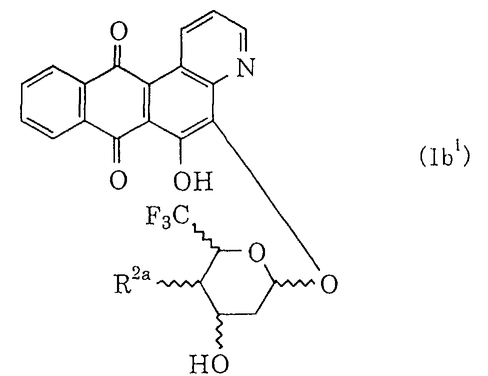 wherein R^{2a} is hydroxyl group, amino group, glycyloxy group, alanyloxy group or acetoxy group.

As examples of the compound of general formula (Ib¹) above, there are given 5-(2,6-dideoxy-6, 6, 6-trifluoro-α-L-lyxo-hexopyranosyloxy)-6-hydroxynaphtho[2,3-f]quinoline-7,12-dione (hereinafter referred sometimes to as Compound L of this invention) represented by the following formula (Ib¹-1) 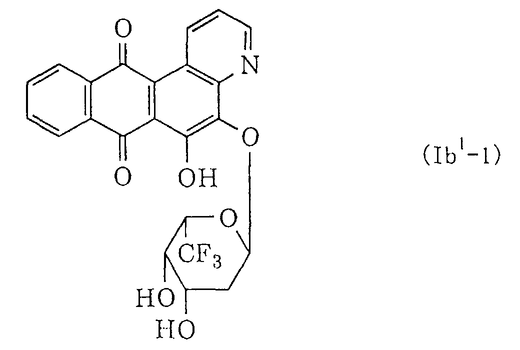 and 5- (2, 6-dideoxy-6, 6, 6-trifluoro-β-L-lyxo-hexopyranosyloxy) -6-hydroxynaphtho[2,3-f]quinoline-7,12-dione (hereinafter referred sometimes to as Compound M of this invention) represented by the following formula (Ib¹-2)

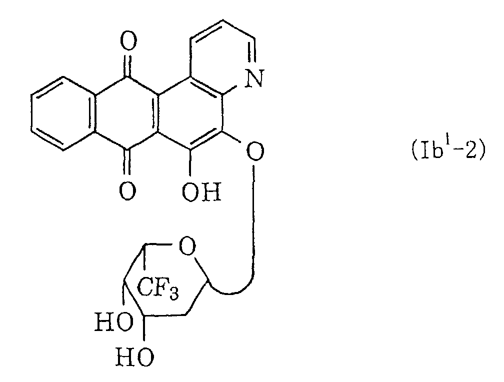

Now, some test examples are given for evaluating the activities of several compounds of general formula (I) according to the first aspect of this invention which are inhibitory against the proliferation of a variety of cancer cells. In Test Example 1 given below, the following five compounds, which are typical examples of the compound of general formula (I) according to the first aspect of this invention, were used as the test compounds.
(1) 5-(2,6-dideoxy-2-fluoro-α-L-talopyranosyloxy)-6-hydroxynaphtho[2,3-f]quinoline-7,12-dione [the compound of formula (Ia¹-1) above; namely, Compound A of this invention hereinbefore given].
(2) 5-(2,6-dideoxy-2-O-methyl-α-L-talopyranosyloxy)-6-hydroxynaphtho[2,3-f]quinoline-7,12-dione [the compound of formula (Ia¹-2) above; namely, Compound B of this invention hereinbefore given].
(3) 5-(2,6-dideoxy-2-fluoro-α-L-galactopyranosyloxy)-6-hydroxynaphtho[2,3-f]quinoline-7,12-dione [the compound of formula (Ia¹-4) above; namely, Compound D of this invention hereinbefore given].
(4) 5-(2,6-dideoxy-2-fluoro-β-L-galactopyranosyloxy)-6-hydroxynaphtho[2,3-f]quinoline-7,12-dione [the compound of formula (Ia¹-5) above; namely, Compound E of this invention hereinbefore given].
(5) 5-(4-amino-2,4,6-trideoxy-2-fluoro-α-L-mannopyranosyloxy)-6-hydroxynaphtho[2,3-f]quinoline-7,12-dione [the compound of formula (Ia²-1) above; namely, Compound F of this invention hereinbefore given]

### Test Example 1

A culture medium for cancer cells was put into test tubes. To the culture medium in each tube, there was transplanted each of such various mouse cancer cells or human cancer cells as shown in Table 1 below. The cells so transplanted were cultivated under the conditions at 37°C in an air atmosphere containing 5 % CO₂. To the medium containing the cancer cells so cultivated, there was added said Compounds A, B, D, E or F of this invention at different concentrations, as the test compound. After the addition of the test compound, the cultivation of the cancer cells was continued for 72 hours, and measurements were made of the concentration of each of the test compounds which was capable of inhibiting by 50 % the proliferation of mouse cancer cells or human cancer cells under test, and which is the 50 % inhibitory concentration:(IC₅₀, µg/ml). As the comparative compound, adriamycin now clinically used was tested to repeat the test in the same way.

The mouse cancer cells and human cancer cells used in the tests above were 12 kinds of cancer cells indicated in Table 1. Namely, they are mouse colon cancer (colon 26), mouse B-cell lymphoma (LB32T), mouse lymphoma (L-1210), human cervical cancer (HeLa), human acute promyeloleukemia (HL60), human prostatic cancer (PC3), human pudendal squamous cell carcinoma (A431), human pulmonary carcinoma (PC6), human mastocarcinoma (MCF7), human esophageal carcinoma (TT), human prostatic cancer (LNcap) and human colon cancer (DLD-1). IC₅₀ values which were measured as the test results are summarily given in Table 1 below.

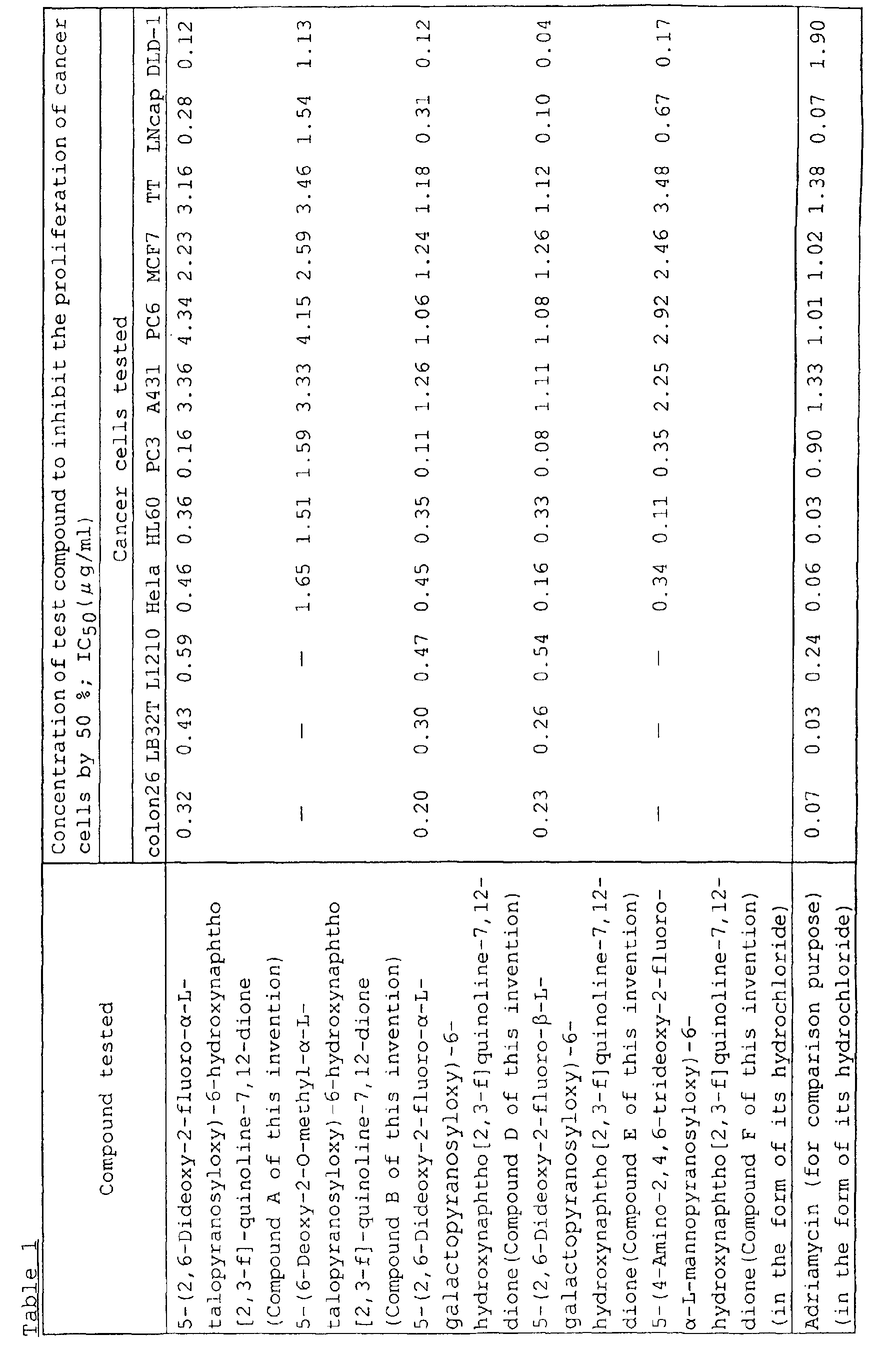

As is clear from the results given in Table 1 above, it has been found that each of Compounds A, B, D, E and F of this invention as tested can inhibit the proliferation of a variety of mouse or human cancer cells at the same level as or at a lower concentration than those of adriamycin, and that the antitumor or anticancer activities of these test compounds are thus the same or a higher level than those of adriamycin. It is particularly noteworthy that Compounds A, D, E and F of this invention have anticancer activities against PC3 which are 5.6 times, 8.2 times, 11 times and 2.6 times, higher than those of adriamycin, respectively, and further that Compounds A, B, D, E and F of this invention have anticancer activities against DLD-1 which are 16 times, 1.7 times, 16 times,48 times and 11 times, higher than those of adriamycin, respectively. It should further be noticeable that such a derivative of Compound A of which 6-hydroxyl group has been methylated, namely 5-(2,6-dideoxy-2-fluoro-α-L-talopyranosyloxy)-6-methoxy-naphtho[2,3-f]-quinoline-7,12-dione, was found to have IC₅₀ value of less than 100 µg/ml, and does not exhibit any appreciable anticancer activity to all human cancer cells as tested. This clearly demonstrates that the presence of the 6-hydroxyl group on the aglycone in the compounds of this invention is essential for the development of the anticancer activities of the compound.

### Test Example 2

In this Test Example, the acute toxicity of Compound A of this invention as administered to mice was evaluated. Thus, Compound A of this invention was intraperitoneally administered to ICR mice (4 mice per group), and then their breeding was continued for 14 days, during which the survival of each mouse was observed to measure the survival day(s) thereof. By way of comparison, similar test was carried out with adriamycin (in the form of hydrochloride) . The results of the tests so obtained are shown in Table 2 below.

**Table 2**

| Dosage (mg/mouse) | Survival day(s) of respective mice | |
|---|---|---|
| | Administration of Compound A of this invention | Administration of adriamycin |
| 4 | >14 (all mice) | |
| 2 | >14 (all mice) | 1,1,1,1 |
| 1 | >14 (all mice) | >14, 6, 2, 2 |
| 0.5 | >14 (all mice) | >14, 6, 6, 2 |
| 0.25 | >14 (all mice) | >14, >14, 7, 6 |
| 0.125 | >14 (all mice) | >14 (all mice) |

As is clear from Table 2 above, the total number of mice of the test mice group have survived for 14 days when Compound A of this invention was administered to them at a dosage of 4 mg/mouse. While, the total number of mice of the test mice group was dead one day after the administration of adriamycin when these mice received adriamycin at a dosage of 2 mg/mouse. When adriamycin was administered to the mice in this Example, the dosage of adriamycin as low as 0.125 mg/mouse was such dosage of adriamycin at which the total number of mice can have survived for 14 days after the administration. From this, it became clear that the toxicity of Compound A of this invention is 1/32 times lower than that of adriamycin.

As is clear from Test Examples 1 and 2 above, the compound of general formula (I) according to this invention exhibit such anticancer activities which are as high as or are superior to those of adriamycin as utilized in clinical treatments. Besides, the compound of general formula (I) has much lower toxicity than that of adriamycin. It is therefore expected that the compound of the formula (I) of this invention is useful for the therapy of a variety of cancers.

Now, the process for the preparation of the compound of general formula (I) according to the first aspect of this invention will be explained.
(A) Of the compound of general formula (I) according to the first aspect of this invention, such compound of general formula (I), where R¹ and/or R² is or are hydroxyl group or amino group, may be prepared by a process comprising condensing the 5-hydroxyl group of 5,6-dihydroxynaphtho[2,3-f]-quinoline-7,12-dione (hereinafter referred sometimes to simply as Alizarin Blue) having by the following formula (A) 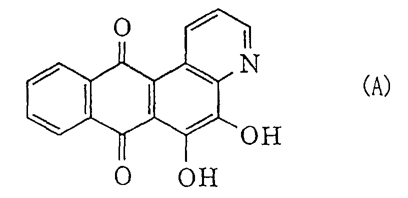 with an L- or D-glycosyl bromide of the following general formula (II) 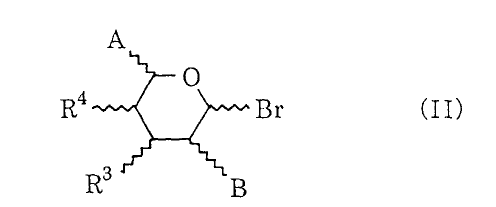 where A and B have the same meanings as defined above, R³ is a hydroxyl group having been protected with an appropriate hydroxy-protecting group, for example, acetyl group, or R³ is an amino group having been protected with an appropriate amino-protecting group, for example, trifluoroacetyl group, or tert-butoxycarbonyl group or benzyloxycarbonyl group, and R⁴ has the same meaning as defined for R³, in an anhydrous organic solvent such as benzene, in the presence of yellow mercuric oxide, mercuric bromide, and Molecular Sieves 3A under reflux, to produce, as the condensation product, an α-or β-glycoside of the following general formula (I') 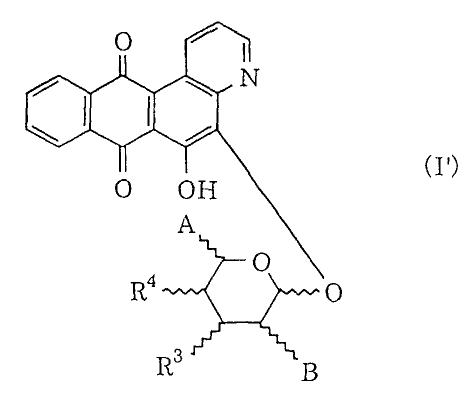 wherein A, B, R³ and R⁴ have the same meanings as defined above, and then eliminating the protecting groups remaining in the groups R³ and/or R⁴, from the resulting condensation product of formula (I') by a conventional deprotecting method, for example, by treatment with sodium methoxide in methanol or by treatment with an aqueous sodium hydroxide solution or by hydrogenolysis.
(B) Of the compound of general formula (I) according to the first aspect of this invention, such compound of general formula (I), where R¹ or R² is a hydroxyl group having been esterified with an α-amino acid residue, may be prepared by a process comprising producing first an α-or β-glycoside of the following general formula (I") 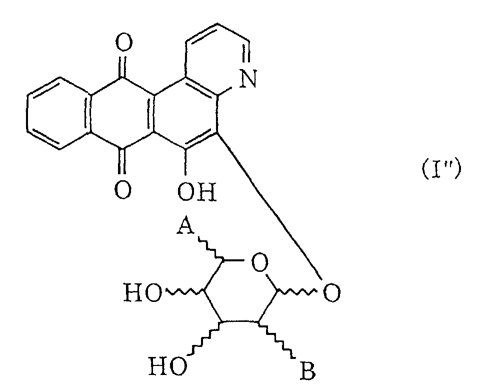 wherein A and B have the same meanings as defined above, by means of the process explained in (A) above, then adding to the glycoside of formula (I")an active ester (such as N-succinimide ester) of an N-protected α-amino acid of the following formula (III) 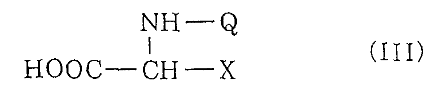 wherein X has the same meaning as defined above and Q is an amino-protecting group, for example, benzyloxycarbonyl group or tert-butoxycarbonyl group, in an amount of equimolar proportion or in a slight excess, stirring the resulting mixture under heating, for example, at an elevated temperature of 50-60 °C to esterify the 3'- or 4'-hydroxyl group of the compound of formula (I") above with the said N-protected amino acid, and finally eliminating the amino-protecting group (Q) from the resulting esterified product by a conventional deprotecting method.
   Upon the reaction of the active ester of the N-protected-α-amino acid of the formula (III) with the compound of formula (I"), there can be obtained a di-ester compound having both the 3'- and 4'-hydroxyl groups of the compound of formula (I") esterified with the N-protected-α-amino acid , when the active ester of an α-amino acid of formula (III) is used in a larger protection. Then, the di-ester compound so produced may be deprotected to give the compound of general formula (I) where both the 3'- and 4'-hydroxyl groups have been esterified with the α-amino acid.
(C) Of the compound of general formula (I) according to the first aspect of this invention, such compound of general formula (I) where R¹ and/or R² is or are a hydroxyl group having been esterified with an acyl group, or is or are a hydroxyl group having been O-glycosylated with a pentose or a hexose, may be prepared by a process comprising reacting the glycoside of formula (I") either with an acyl bromide of the following formula (IV) 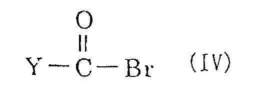 wherein Y has the same meaning as defined above, in an organic solvent in the presence of an acid-binding agent, for example, sodium hydroxide, or with a pentosyl bromide or hexosyl bromide in the presence of a condensing agent, for example, mercuric cyanide.

It is considered that the novel Alizarin Blue derivative having the general formula (I) according to the first aspect of this invention have high anticancer activities and low toxicities and thus are expected to be very useful as an anticancer agent and to be useful in the therapy of a variety of cancers similarly to adriamycin. Therefore, the compound of the general formula (I) according to the first aspect of this invention is valuably usable as a therapeutic anticancer agent in therapeutic treatments of solid cancers and ascitic cancers.

According to a second aspect of this invention, therefore, there is provided an anticancer or antitumor composition comprising as an active ingredient a 5-glycosyloxy -6-hydroxynaphtho[2,3-f]quinoline-7, 12-dione of the general formula (I) above, or a pharmaceutically acceptable acid addition salt thereof, in combination with a pharmaceutically acceptable solid or liquid carrier.

The compound of general formula (I) as active ingredient in the composition of the second aspect of this invention may be a 5-glycosyloxy-6-hydroxynaphtho[2,3-f]quinoline-7,12-dione of the general formula (Ia) or general formula (Ib) above, or an acid addition salt thereof.

As explained in item (B) given above, such compound of general formula (I) according to the first aspect of this invention, where R¹ or R² or both of them stand for a hydroxyl group having been esterified with an á-amino acid residue, may be prepared by the process comprising esterifying the 3'-and/or 4'-hydroxyl group(s) of such á- or â-glycoside compound of the general formula (I )with an N-protected á-amino acid of the formula (III) above. The compound of general formula (I) where R¹ and R² each stand for a hydroxyl group and a hydroxyl group remains at the 6-position of the aglycone moiety of the compound, including a compound of general formula (Ia¹) and a compound of formula (Ib¹-1) or a compound of formula (Ib¹-2) above, may be a 6-hydroxynaphtho[2,3-f]quinoline-7,12-dione derivative represented by general formula (I ) 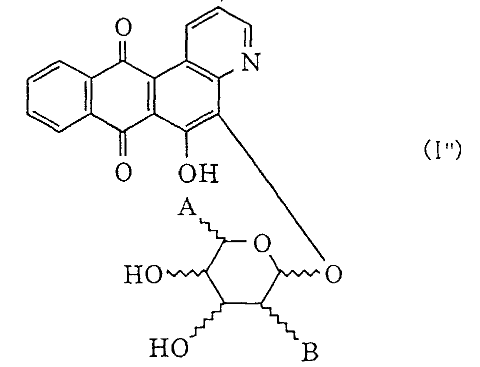 wherein A is methyl group or CF₃ group, and B is hydrogen atom or an electron-withdrawing group as above. The compound of general formula (I ) is, in general, sparlingly soluble or insoluble in water. When the compound of general formula (I) is administered by intravenous injection, there is a possibility that a trouble occurs in the intravenous administration due to the difficulty of the compound to dissolve in blood plasma. While, according to the process for the preparation described in item (B) above, it is feasible to obtain such a compound of the general formula (Ia⁴) which have an improved solubility in water owing to the 3'- and/or 4'-hydroxyl group(s) of the compound of general formula (I ) that have been esterified with an á-amino acid of formula (III).

According to our recent another investigation, we have now found that when use is made of an ù-amino acid in place of an á-amino acid of the above formula (III) in order to esterify the 3'- and/or 4'-hydroxyl group(s) of the compound of general formula (I ), and occasionally also to esterify the 6-hydroxyl group of the aglycone moiety of the compound of general formula (I ) with said ω-amino acid, and when the resulting esterified product is optionally further treated so as to convert the terminal amino group of the introduced amino acid chain into a salt thereof by an addition-reaction with an acid, it is feasible to produce from the compounds of general formula (I ) such a water-soluble derivative which can maintain the anticancer activities and has been imparted with an improved solubility in water.

According to a third aspect of this invention, therefore, there is provided a 5-L or D-glycosyloxy-6-hydroxy or 6-O-substituted hydroxynaphtho[2,3-f]quinoline-7,12-dione represented by the following general formula (V) 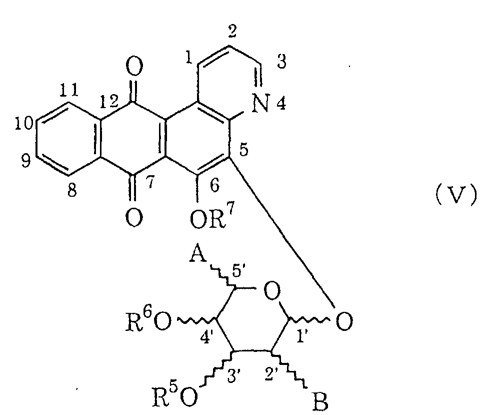 wherein A is methyl group or trifluoromethyl group, and B is hydrogen atom, or an electron-withdrawing group chosen from fluoro group, chloro group, bromo group, iodo group, difluoro group, an alkoxy group of 1-5 carbon atoms and cyano group, provided that B stands for hydrogen atom when A is trifluoromethyl group and that B stands for the electron-withdrawing group when A is methyl group, and R⁵ is hydrogen atom or an ω-amino acid residue of the following formula (d)

-CO-G-NH₂ (d)

where G is a straight-chain alkylene group of 2-6 carbon atoms which may further be substituted by a (C₁-C₆) alkyl group, and R⁶ and R⁷ each have the same meanings as defined for R⁵, provided that all of R⁵, R⁶ and R⁷ do not represent hydrogen atoms, simultaneously, or a pharmaceutically acceptable salt thereof.

The compound of general formula (V) according to the third aspect of this invention may preferably be a 5-(2,6-dideoxy-2-fluoro-3-mono-O-aminoalkanoyl- or 4-mono-O-aminoalkanoyl-or 3,4-di-O-aminoalkanoyl-L or D-talopyranosyloxy or galactopyranosyloxy or mannopyranosyloxy)-6-hydroxy or 6-aminoalkanoyloxynaphtho[[2,3-f]quinoline-7,12-dione represented by the following general formula (Va) 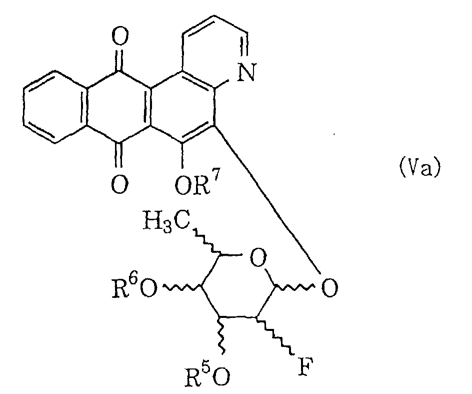 wherein each of R⁵, R⁶ and R⁷ is either an ω-amino acid residue of formula (d) having the same meaning as defined in the above formula (V), or is hydrogen atom, provided that all of R⁵, R⁶ and R⁷ do not represent hydrogen atoms, simultaneously.

The above-mentioned ω-amino acid residue may preferably be 3-amino-propionyl group, 4-aminobutylyl group, 5-aminopentanoyl group, 6-aminohexanoyl group, 7-aminoheptanoyl group, 4-amino-2-methylbutylyl group or 5-amino-3-ethylpentanoyl group.

The compound of the above general formula (Va), in preferred embodiments thereof, includes a compound of general formula (Va-1), general formula (Va-2) and general formula (Va-3) as given below.
(1) 5-(2,6-Dideoxy-2-fluoro-3-mono-O-aminoalkanoyl- or 4-mono-O-aminoalkanoyl- or 3,4-di-O-aminoalkanoyl-L or D-talopyranosyloxy)-6-hydroxynaphtho[2,3-f]quinoline-7,12-dione represented by general formula (Va-1) 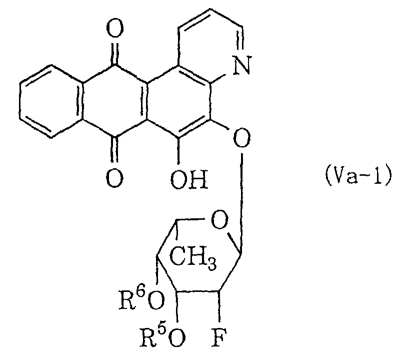 wherein R⁵ is hydrogen atom or an ω-amino acid residue of formula (d) shown in the formula (V), and R⁶ has the same meaning as defined for R⁵, provided that R⁵ and R⁶ simultaneously do not represent hydrogen atom.
(2) 5-(2,6-Dideoxy-2-fluoro-3-mono-O-aminoalkanoyl- or 4-mono-O-aminoalkanoyl- or 3,4-di-O-aminoalkanoyl-L or D-galactopyranosyloxy)-6-hydroxy or 6-aminoalkanoyloxynaphtho[2,3-f]quinoline-7,12-dione represented by general formula (Va-2) 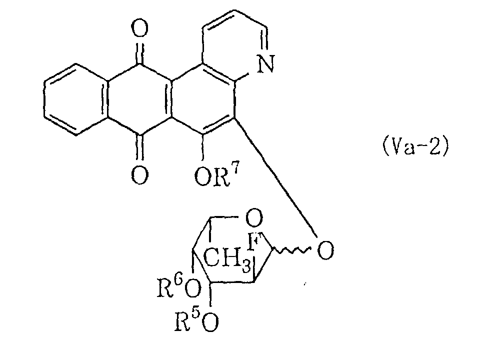 wherein R⁵ is hydrogen atom or an ω-amino acid residue of formula (d) defined in the above formula (V), and R⁶ and R⁷ each have the same meanings as defined for R⁵, provided that R⁵' R⁶ and R⁷ simultaneously do not represent hydrogen atom.
(3) 5-(2, 6-Dideoxy-2-fluoro-3-mono-O-aminoalkanoyl- or 4-mono-O-aminoalkanoyl- or 3,4-di-O-aminoalkanoyl-L or D-mannopyranosyloxy)-6-hydroxynaphtho[2,3-f]quinoline-7,12-dione represented by general formula (Va-3) 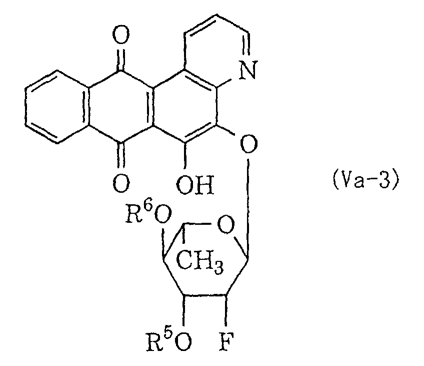 wherein R⁵ is hydrogen atom or an ω-amino acid residue of formula (d) defined in the above formula (V), and R⁶ has the same meaning as defined for R⁵, provided that R⁵ and R⁶ simultaneously do not represent hydrogen atom.

As preferred examples of the compound of the general formula (Va-1) above, there may be listed the following three compounds.
(1) 5-[3-O-(3-aminopropionyl)-2, 6-dideoxy-2-fluoro-α-L-talopyranosyloxy]-6-hydroxynaphtho[2,3-f]quinoline-7,12-dione (Compound N of this invention)
(2) 5-[4-O-(3-aminopropionyl)-2,6-dideoxy-2-fluoro-α-L-talopyranosyloxy]-6-hydroxynaphtho[2,3-f]quinoline-7,12-dione (Compound O of this invention)
(3) 5-(3,4-Di-O-(3-aminopropionyl)-2,6-dideoxy-2-fluoro-α-L-talopyranosyloxy]-6-hydroxynaphtho[2,3-f] quinoline-7,12-dione (Compound P of this invention)

As preferred examples of the compound of the general formula (Va-2) above, there may be listed the following four compounds.
(1) 5-[3,4-di-O-(3-aminopropionyl)-2,6-dideoxy-2-fluoro-α-L-galactopyranosyloxy]-6-hydroxynaphtho[2,3-f] quinoline-7,12-dione (Compound Q of this invention)
(2) 5-[3-O-(3-aminopropionyl)-2,6-dideoxy-2-fluoro-β-L-galactopyranosyloxy]-6-hydroxynaphtho[2,3-f]quinoline-7,12-dione (Compound R of this invention)
(3) 5-[3,4-di-O-(3-aminopropionyl)-2,6-dideoxy-2-fluoro-β-L-galactopyranosyloxy]-6-hydroxynaphtho[2,3-f] quinoline-7,12-dione (Compound S of this invention)
(4) 5-[3,4-di-O-(3-aminopropionyl)-2,6-dideoxy-2-fluoro-β-L-galactopyranosyloxy]-6-(3-aminopropionyl) oxynaphtho[2,3-f]quinoline-7,12-dione (Compound T of this invention)

All of the seven Compounds N to T of this invention as above-mentioned have such good water-solubilities which are higher than 20 mg/ml at 25 °C.

The synthesis of the compound of general formula (V), including the compound of general formula (Va), can be effected by a process comprising reacting a compound of the above general formula (I") with an ω-amino acid of the following general formula (VI)

HOOC-G-NH-Q (VI)

wherein G is a straight-chain alkylene group of 2-6 carbon atoms which may further be substituted by a (C₁-C₆) alkyl group, and Q is an amino-protecting group, for example, benzyloxycarbonyl group or t-butoxycarbonyl group, or an active ester thereof, in dichloromethane in the presence of 1-ethyl-3-(3-dimethylaminopropyl)carbodiimide hydrochloride and 4-dimethylaminopyridine at a temperature, for example, at room temperature or higher, to mono-esterify the 3'- or 4'-hydroxyl group of the compound of general formula (I") with the N-protected-ω-amino acid of the formula (VI), and then eliminating the amino-protecting group (Q) from the resulting mono-esterified product by a conventional method.

Further, when the reaction of the N-protected-ω-amino acid of formula (VI) with the compound of formula (I") is carried out in such a way that use is made of a larger propertion of the ω-amino acid of formula (VI), it is feasible to produce such di-ester product in which both the 3'- and 4'-hydroxyl groups of the compound of formula (I") have been esterified with the N-protected-ω-amino acid. By effecting a subsequent deprotection of the resulting di-ester product to eliminate the amino-protected group (Q), there can be obtained such a compound of the general formula (V) or (Va) where both the 3'- and 4'-hydroxyl groups have been esterified with the ω-amino acid.

As preferred examples of the ω-amino acids of formula (VI), there may be listed 3-aminopropionic acid, 4-aminobutyric acid, 5-aminovaleric acid, 6-aminohexanoic acid, 7-aminoheptanoic acid, 4-amino-2-methyl-butyric acid and 5-amino-3-ethylvaleric acid.

Now, the compound of general formula (V) according to the third aspect of this invention, specifically Compounds N to T ( seven compounds in total), which are typical examples of compounds of general formula (Va), have been tested in the following Test Example 3 to evaluate their activities inhibitory against the proliferation of a variety of cancer cells of mice and human.

### Test Example 3

The test method and the nature of cancer cells to be tested are the same as those in Test Example 1 given hereinbefore. In this Test Example 3, however, eight kinds of cancer cells indicated in Table 3 below are used, among the twelve kinds of cancer cells which were used in Test Example 1.

As the test results of measurement, the evaluated values of the concentration of each test compound which could inhibit the proliferation of cancer cells by 50 % (namely, 50 % inhibitory concentration:IC₅₀, µg/ml) are given in Table 3. By way of comparison, similar test was made with adriamycin, and IC₅₀ values of adriamycin are also given in Table 3 below.

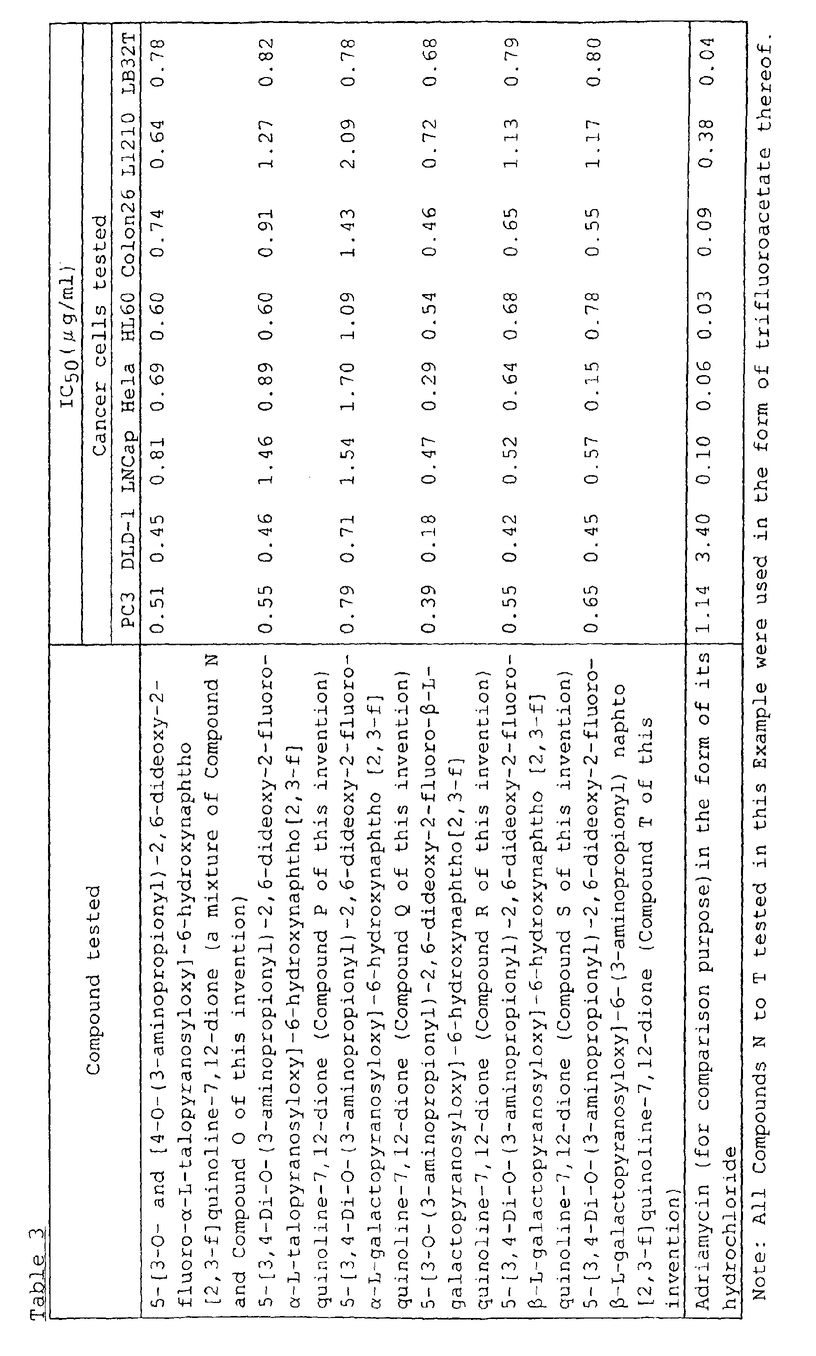

As is clear from Table 3, it is observed that each of Compounds N, O, P, Q, R, S and T of this invention has such activities inhibitory against the proliferation of cancer cells, to an extent that the compound is effective against the proliferation of human cancer cells at a concentration of lower than that of adriamycin, and that each of Compounds N to T shows a lower inhibitory activity against the proliferation of various mouse cancer cells than that of adriamycin. It is noteworthy that all tested Compounds of this invention exhibit strong anticancer activities against human prostatic cancer PC3 and human colon cancer DLD-1, and particularly that Compound R of this invention exhibits such anticancer activity against PC3 which is 2.9 times higher than that of adriamycin, and also that Compound R exhibits such anticancer activity against DLD-1 which is 19 times higher than that of adriamycin.

The compound of general formula (V) according to the third aspect of this invention, particularly the compound of general formula (Va), is useful as an anticancer agent because of its excellent anticancer activities as are clearly demonstrated in Test Example 3.

According to a fourth aspect of this invention, therefore, there is provided an anticancer or antitumor composition comprising a 5-L or D-glycosyloxy-6-hydroxy or 6-O-substituted hydroxynaphtho[2,3-f]quinoline-7,12-dione of the general formula (V) or a pharmaceutically acceptable acid addition salt thereof, as an active ingredient, in combination with a pharmaceutically acceptable solid or liquid carrier or carriers.

In the composition according to the fourth aspect of this invention, the active compound to be incorporated may be a compound of general formula (Va-1), a compound of general formula (Va-2) or a compound of general formula (Va-3) as given above, or an acid addition salt thereof.

When the compound of general formula (I) according to the first aspect of this invention or the compound of general formula (V) according to the third aspect of this invention is administered in its practical applications, the compound may generally be administered orally or parenterally. The compound of general formula (I) or general formula(V) of this invention may also be administered orally or parenterally, in the form of a composition such as powder, granules, tablets, syrup or injectable preparation which may be prepared by blending the active compound with a solid carrier such as starch, or a liquid carrier such as ethanol.

As general means for administration of the compound of this invention, there may be mentioned intraperitoneal injections, subcutaneous injections, intravascular injections such as intraveneous and intra-arterial injections, and topical injections for animals; as well as intravascular injections such as intravenous and intra-arterial injections, and topical injections for human. The dosage of the compound may be determined depending upon the results of animal tests and various conditions, and the compound may be administered continuously or intermittently within a certain limited range of a total administration dosage.

However, it is needles to say that the dosage of the compound of general formula (I) according to the first aspect of this invention, or the compound of general formula (V) according to the third aspect of this invention to be administered may vary properly, depending upon the kind of cancer or tumor to be treated, the method for administration of the compound to be administered, the conditions of patient or animals to be treated, for example, age, body weight, sex, sensitivity, diet, time of administration, kind of medicine as used together, level or degree of cancer or tumor, and so on. The ordinary dosage of the compound of this invention to be used as anticancer agent may be the same level as that of adriamycin. Appropriate dosage and frequency of administration of the compound of this invention to be given under certain specific conditions have to be determined on the basis of the guideline above-mentioned, in view of such tests for determining an appropriate dosage of the compound which are made by a medical specialist. The aforesaid conditions for the administration of the compound are taken into consideration similarly, upon the oral administration and parenteral administration of the compound.

### BEST MODE FOR CARRYING OUT THE INVENTION

This invention is now concretely explained with referance to the following Examples 1-10 which illustrate the preparation of the compound of general formula (I) according to the first aspect of this invention. However, this invention is in no way limited to the Examples shown below.

### Example 1

### (1) Preparation of 5-(3,4-di-O-acetyl-2,6-dideoxy-2-fluoro-a-L-talopyranosyloxy)-6-hydroxynaphtho[2,3-f] quinoline-7,12-dione (Compound 2)

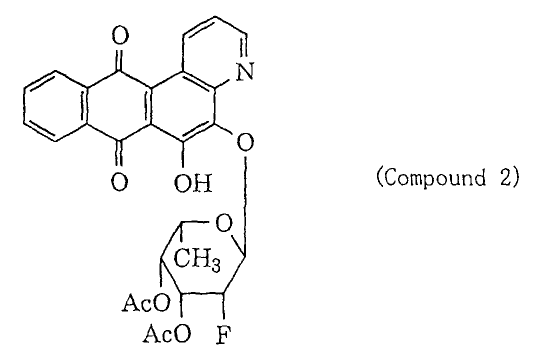

In anhydrous benzene (31.5 ml), there were suspended 3,4-di-O-acetyl-2,6-dideoxy-2-fluoro-α-L-talopyranosyl bromide (Compound 1) (271 mg) [refer to K. Ok, Y. Takagi, T. Tsuchiya, S. Umezawa and H. Umezawa, "Carbohydrate Research", 169, pp.69-81, 1987], Alizarin Blue (210 mg), yellow mercuric oxide (703mg), mercuricbromide (208mg) and powdery Molecular Sieves 3A (2.1 g). The resulting suspension was refluxed in a dark place for 5 hours under stirring.

The reaction solution so obtained was filtered through a layer of Celite to obtain the filtrate. The Celite was washed with benzene. The washings so obtained were combined with said filtrate, and the solution as combined was washed with a 30 % aqueous potassium iodide solution and then with water. The solution so washed was dried over anhydrous sodium sulfate and then concentrated under a reduced pressure. The resulting residue was treated, for the isolation and purification of the intended product, by a silica gel preparative TLC (developing solvent: chloroform-ethyl acetate, 12:1). The titled Compound 2 of this invention was obtained as red solid (222 mg, yield 58 %).
[α]_{D}²⁴ +60° (c 0.05, chloroform)
¹H-NMR spectrum (in deuterochloroform)
δ 2.16 (3H, s, OAc)
2.19 (3H, s, OAc)
5.21 (1H, br d, H-2')
6.29 (1H, dd, J_{1',2'} = 1, J_{1',F} = 9Hz, H-1')
7.45 (1H, dd, H-2)
8.91 (1H, dd, H-3)
9.82 (1H, dd, H-1)
12.73 (1H, s, OH)

¹⁹F-NMR spectrum (in deuterochloroform, CFCl₃ as internal standard)
δ -201.9

| | | | | |
|---|---|---|---|---|
| Elemental analysis (for C₂₇H₂₂FNO₉) | | | | |
| Calculated | C,61.94; | H, 4.24; | F, 3.63; | N, 2.68% |
| Found | C, 61.67; | H, 4.23; | F, 3.66; | N, 2.67% |

### (2) Preparation of 5-(2,6-dideoxy-2-fluoro-α-L-talopyranosyloxy)-6-hydroxynaphtho[2,3-f]quinoline-7,12-dione of the following formula (Ia¹-1) (Compound A of this invention)

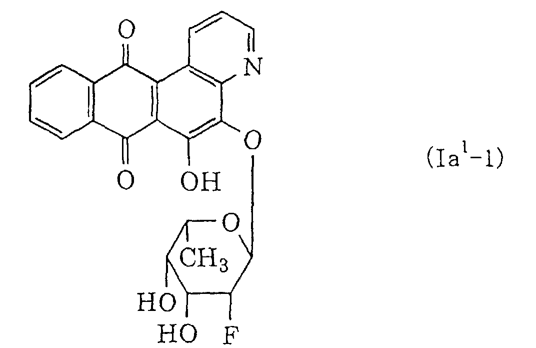

Compound 2 as obtained in Example 1-(1), namely 5-(3,4-di-O-acetyl-2,6-dideoxy-2-fluoro-α-L-talopyranosyloxy)-6-hydroxynaphtho[2,3-f]quinoline-7,12-dione (175 mg), was dissolved in a mixture of tetrahydrofuran (17.5 ml) and methanol (8.8 ml). To the resulting solution was added a methanolic solution of 0.5 M sodium methoxide (0.14 ml). The resulting mixture was allowed to stand for 15 hours, and was then added with a methanolic solution of 0.5 M sodium methoxide (0.14 ml). The resulting mixture was allowed to stand for 25 hours and then added further with a methanolic solution of 0.5 M sodium methoxide (0.14 ml). The mixture so obtained was allowed to stand for 18 hours, for effecting the deprotection reaction to eliminate the 3,4-di-O-acetyl group of Compound 2.

Dry ice (solidified carbon dioxide) was added to the resulting reaction solution, and the resultant mixture was concentrated under a reduced pressure. The resulting residue was then dissolved in a mixture of chloroform-methanol (5:1). The resulting solution was washed with water and the remaining organic layer was concentrated under a reduced pressure. The resulting residue was dissolved in a small volume of a mixture of chloroform-methanol (2:1). To the solution as obtained was added isopropylether to cause reprecipitation of the target product, whereby the titled Compound A of this invention was afforded as red solid (124 mg, yield 77 %).
[α]_{D}²⁴ +10° (c, 0.1, pyridine)
¹H-NMR spectrum (in deuteropyridine)
δ 5.74 (1H, br d, H-2')
6.89 (1H, br d, H-1')
7.41 (1H, dd, H-2)
9.00 (1H, br d, H-3)
9.96 (1H, br d, H-1)

¹⁹F-NMR spectrum (in deuteropyridine, CFCl₃ as internal standard)
δ -199.9 (ddd)

| | | | | |
|---|---|---|---|---|
| Elemental analysis (for C₂₃H₁₈FNO₇ · 0.5H₂O) | | | | |
| Calculated | C, 61.60; | H, 4.27; | F, 4.24; | N, 3.12% |
| Found | C, 61.85; | H, 4.08; | F, 4.40; | N, 3.20% |

### Example 2

### (1) Preparation of 5-(3,4-di-O-acetyl-6-deoxy-2-O-methyl-α-L-talopyranosyloxy)-6-hydroxynaphtho[2,3-] quinoline-7,12-dione (Compound 4)

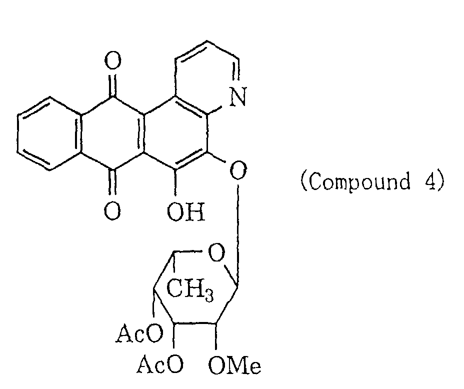

In anhydrous benzene (3.8 ml), there were suspended 3,4-di-O-acetyl-6-deoxy-2-O-methyl-α-L-talopyranosyl bromide (Compound 3) (24.2mg) [refer to Y. Takagi, N. Kobayashi, T. Tsuchiya, S. Umezawa, T. Takeuchi, K. Komuro and C. Nosaka, "The Journal of Antibiotics", 42, pp.1318-1320, 1989], Alizarin Blue (20.4 mg), yellow mercuric oxide (72.5 mg), mercuric bromide (24.6 mg) and powdery Molecular Sieves 3A (193 mg). The resulting suspension was refluxed in a dark place under stirring for 5 hours.

The reaction solution so obtained was filtered through a layer of Celite to obtain the filtrate. The Celite was washed with benzene. The washings so given were combined with said filtrate, and the solution as combined was washed with a 30 % aqueous potassium iodide solution and then with water. The solution so washed was dried over anhydrous sodium sulfate and then concentrated under a reduced pressure. The resulting residue was subjected to a flash silica gel column chromatography (developing solvent: chloroform-ethyl acetate, 12:1) for the isolation and purification of the intended product. The titled Compound 4 of this invention was obtained as red solid (14.6 mg, yield 39 %).
[α]_{D}²⁰ -6° (c 0.05, chloroform)
¹H-NMR spectrum (in deuterochloroform)
δ 2.14 (3H, s, OAc)
2.20 (3H, s, OAc)
3.66 (3H, s, OMe)
6.27 (1H, d, H-1')
7.49 (1H, dd, H-2)
8.94 (1H, dd, H-3)
9.91 (1H, dd, H-1)
12.85 (1H, s, OH)

| | | | |
|---|---|---|---|
| Elemental analysis (for C₂₈H₂₅NO₁₀ · H₂O) | | | |
| Calculated | C, 60.76; | H, 4.92; | N, 2.53% |
| Found | C, 60.91; | H, 4.74; | N, 2.53% |

### (2) Preparation of 5-(6-deoxy-2-O-methyl-α-L-talopyranosyloxy)-6-hydroxynaphtho[2,3-f]quinoline-7,12-dione of the following formula (Ia¹-2) (Compound B of this invention)

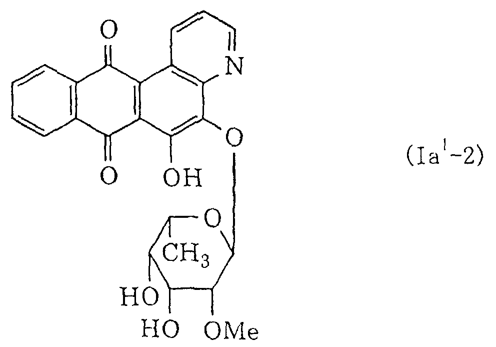

Compound 4 as obtained in Example 2-(1), namely 5-(3,4-di-O-acetyl-6-deoxy-2-O-methyl-α-L-talopyranosyloxy)-6-hydroxynaphtho[2,3-f]quinoline-7,12-dione (6.9 mg), was dissolved in a mixture of tetrahydrofuran (0.7 ml) and methanol (0.7 ml). To the resulting solution was added a 5 M sodium methoxide-methanol solution (0.01 ml). The resulting mixture was allowed to stand for 15 hours, and then further added with a 5 M sodium methoxide solution in methanol (0.02 ml). The resulting mixture was allowed to stand for 18 hours, for effecting the deprotection reaction to eliminate the acetyl group from Compound 4.

Dry ice was added to the resulting reaction solution, and the mixture so obtained was concentrated under a reduced pressure. The resulting residue was dissolved in a mixture of chloroform-methanol (5:1). The resulting solution was washed with water, and the remaining organic layer was concentrated under a reduced pressure. The resulting residue was dissolved in a small volume of a mixture of chloroform-methanol (2:1).To the solution as obtained were added isopropylether and hexane to cause reprecipitation of the target product. The titled Compound B of this invention was yielded as red solid (4.1 mg, yield 71 %).
[α]_{D}²² +56° (c, 0.025, chloroform)
¹H-NMR spectrum (in deuterochloroform)
δ 3.64 (3H, s, OMe)
6.31 (1H, br s, H-1')
7.50 (1H, dd, H-2)
8.95 (1H, br d, H-3)
9.93 (1H, d, H-1)

| | | | | |
|---|---|---|---|---|
| Elemental analysis (for C₂₄H₂₁NO₈ · 0.5H₂O) | | | | |
| Calculated | C, 62.61; | H, 4.82; | N, 3.04% | |
| Found | C, 62.56; | H, 4.84; | N, 3.08% | |

### Example 3

### (1) Preparation of 5-(3,4-di-O-acetyl-2,6-dideoxy-2-fluoro-α-L-mannopyranosyloxy)-6-hydroxynaphtho[2,3-f] quinoline-7,12-dione (Compound 6)

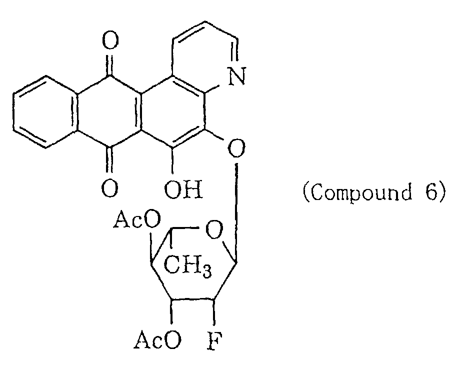

In anhydrous benzene (6.5 ml), there were suspended 3,4-di-O-acetyl-2,6-dideoxy-2-fluoro-α-L-mannopyranosyl bromide (Compound 5) (54 mg) [refer to Y. Takagi, T. Tsuchiya, T. Miyake, T. Takeuchi, and S. Umezawa, a Japanese journal "The Journal of Organic Synthetic Chemical Association, 50, pp.131-142, 1992], Alizarin Blue (42 mg), yellow mercuric oxide (141 mg), mercuric bromide (42 mg) and powdery Molecular Sieves 3A (420 mg). The resulting suspension was refluxed in a dark place under stirring for 6 hours.

The reaction solution so obtained was filtered through Celite to obtain the filtrate. The Celite was washed with benzene. The washings so given were combined with said filtrate, and the combined solution was washed with a 30 % aqueous potassium iodide solution and then with water. The solution so washed was then dried over anhydrous sodium sulfate and concentrated under a reduced pressure. The resulting residue was subjected to a flash silica gel column chromatography (developing solvent: chloroform-ethyl acetate, 15:1) for the isolation and purification of the intended product. The titled Compound 6 of this invention was obtained as red solid (38.5 mg, yield 51 %).
¹H-NMR spectrum (in deuterochloroform)
   δ 2.18 (3H, s, OAc)
   2.20 (3H, s, OAc)
   5.27(1H, br d, H-2')
   6.27 (1H, dd, J_{1',2'} = 1, J_{1',F} = 8.5Hz, H-1')
   7.44 (1H, dd, H-2)
   8.93 (1H, dd, H-3)
   9.80 (1H, dd, H-1)
   12.81 (1H, s, OH)
¹⁹F-NMR spectrum (in deuterochloroform, CFCl₃ as internal standard)
   δ -202.3 (ddd)

### (2) Preparation of 5-(2,6-dideoxy-2-fluoro-α-L-mannopyranosyloxy)-6-hydroxynaphtho[2,3-f]quinoline-7,12-dione of the following formula (Ia¹-3) (Compound C of this invention)

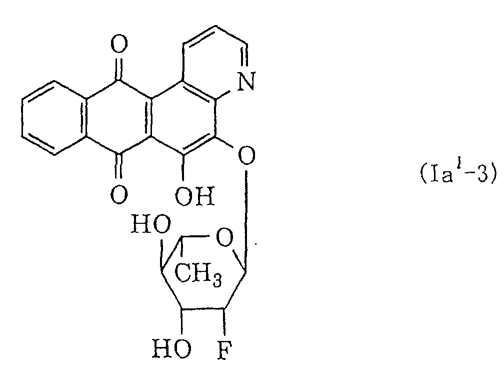

Compound 6 as obtained in Example 3-(1), namely 5-(3,4 -di-O-acetyl-2,6-dideoxy-2-fluoro-α-L-mannopyranosyloxy)-6-hydroxynaphtho[2,3-f]quinoline-7,12-dione (28 mg), was dissolved in a mixture of tetrahydrofuran (2.8 ml) and methanol (1.4 ml). To the resulting solution was added a 0.5 M sodium methoxide solution in mothanol (0.07 ml). The resulting mixture was allowed to stand for 18 hours, for effecting the deprotection reaction to eliminate the acetyl group from Compound 6.

Dry ice was added to the resulting reaction solution, and the mixture so obtained was concentrated under a reduced pressure. The resulting residue was dissolved in a mixture of chloroform-methanol (5:1). The resulting solution was washed with water and the remaining organic layer was concentrated under a reduced pressure. The resulting residue was dissolved in a small amount of a mixture of chloroform-methanol (2:1). To the solution was added isopropylether to cause reprecipitation of the target product, whereby the titled Compound C of this invention was afforded as red solid(19 mg, yield 82 %).
¹H-NMR spectrum (in deuteropyridine)
   δ 5.70 (1H, br d, H-2')
   6.85 (1H, br d, H-1')
   7.39 (1H, dd, H-2)
   9.05 (1H, br d, H-3)
   9.91 (1H, br d, H-1)
¹⁹F-NMR spectrum (in deuteropyridine, CFCl₃ as internal standard)
   δ -198.1 (ddd)

### Example 4

### (1) Preparation of 5-(3,4-di-O-acetyl-2,6-dideoxy-6,6,6-trifluoro-α-L-lyxo-hexopyranosyloxy)-6-hydroxynaphtho[2,3 -f]quinoline-7,12-dione(Compound 8a) and 5-(3,4-di-O-acetyl -2, 6-dideoxy-6, 6, 6-trifluoro-β-L-lyxo-hexopyranosyloxy)-6-hydroxynaphtho[2,3-f]quinoline-7,12-dione (Compound 8b)

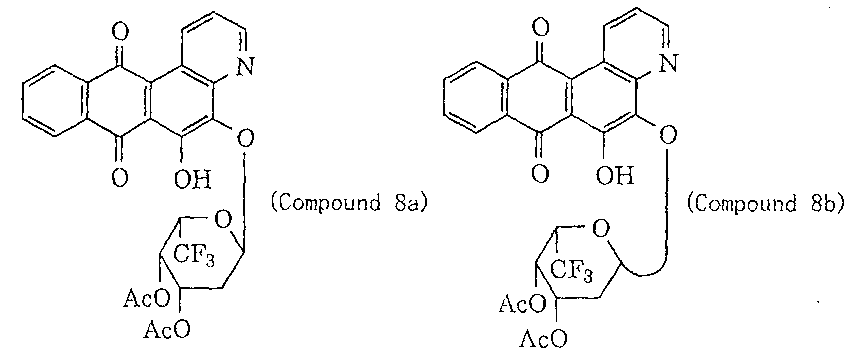

In anhydrous benzene (6.6 ml), there were suspended 3,4-di-O-acetyl-2,6-dideoxy-6,6,6-trifluoro-α-L-lyxo-hexopyranosyl bromide (Compound 7) (63mg) [refer to Y. Takagi, K. Nakai, T. Tsuchiya and T. Takeuchi, "Journal of Medicinal Chemistry", 39, pp.1582-1588, 1996], Alizarin Blue (44 mg), yellow mercuric oxide (147 mg), mercuric bromide (44 mg) and powdery Molecular Sieves 3A (450 mg). The resulting suspension was refluxed in a dark place under stirring for 5 hours.

The reaction solution so obtained was filtered through Celite to obtain the filtrate. The Celite was washed with benzene. The washings so given were combined with said filtrate, and the combined solution was washed with a 30 % aqueous potassium iodide solution and then with water. The solution so washed was dried over anhydrous sodium sulfate and then concentrated under a reduced pressure. The resulting residue was subjected to a flash silica gel column chromatography (developing solvent: chloroform-ethyl acetate, 12:1) for the isolation and purification of the intended product. The titled Compound 8a (27 mg, yield 32 %)and the titled Compound 8b (25 mg, yield 29 %) were obtained as red solid, respectively.

### Physical properties of Compound 8a

¹H-NMR spectrum (in deuterochloroform)
   δ 1.99 (3H, s, OAc)
   2.16 (3H, s, OAc)
   5.85 (1H, br d, J = 3.5Hz, H-1')
   7.50 (1H, dd, H-2)
   8.87 (1H, dd, H-3)
   9.81 (1H, dd, H-1)
   12.56 (1H, s, OH)
¹⁹F-NMR spectrum (in deuterochloroform, CFCl₃ as internal standard)
   δ -74.3 (d)

### Physical properties of Compound 8b

¹H-NMR spectrum (in deuterochloroform)
   δ 2.01 (3H, s, OAc)
   2.09 (3H, s, OAc)
   5.22 (1H, dd, J = 10 and 3Hz, H-1')
   7.48 (1H, dd, H-2)
   8.89 (1H, dd, H-3)
   9.85 (1H, dd, H-1)
   12.51 (1H, s, OH)
¹⁹F-NMR spectrum (in deuterochloroform, CFCl₃ as internal standard)
   δ -74.0 (d)

### (2) Preparation of 5-(2,6-dideoxy-6,6,6-trifluoro-α-L-lyxo-hexo-pyranosyloxy)-6-hydroxynaphtho[2,3-f]quinoline-7,12-dione of the following formula (Ib¹-1) (Compound L of this invention)

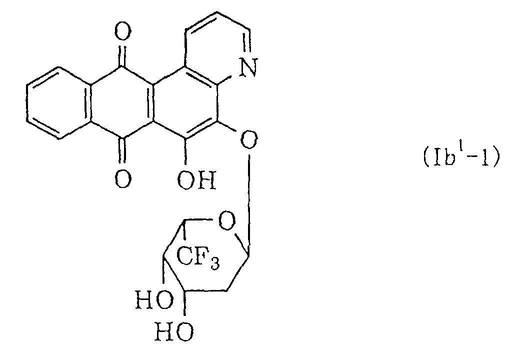

Compound 8a as obtained in Example 4-(1), namely 5-(3,4-di-O-acetyl-2,6-dideoxy-6,6,6-trifluoro-α-L-lyxo-hexopyranosyloxy)-6-hydroxynaphtho[2,3-f]quinoline-7,12-dione (22 mg), was dissolved in a mixture of tetrahydrofuran (2.4 ml) and methanol (1.2 ml). To the resulting solution was added 0.5 M sodium methoxide-methanol solution (0.03 ml). The resulting mixture was allowed to stand for 15 hours and was then added with a further amount of 0.5 M sodium methoxide-methanol solution(0.03 ml). The mixture so obtained was allowed to stand for 16 hours, for effecting the deprotection reaction to eliminate the acetyl group from Compound 8a.

Dry ice was added to the resulting reaction solution and the mixture so obtained was concentrated under a reduced pressure. The resulting residue was dissolved in a mixture of chloroform-methanol (5:1). The resulting solution was washed with water and the remaining organic layer was concentrated under a reduced pressure. The resulting residue was dissolved in a small volume of a mixture of chloroform-methanol (3:1). To the solution as obtained was added isopropylether to cause reprecipitation of the target product, whereby the titled Compound L of this invention was afforded as red solid(13 mg, yield 70 %).
¹H-NMR spectrum (in deuteropyridine)
   δ 6.05(1H, br d, H-1')
   7.39 (1H, dd, H-2)
   9.02 (1H, br d, H-3)
   9.93 (1H, br d, H-1)
¹⁹F-NMR spectrum ( in deuteropyridine, CFCl₃ as internal standard)
   δ -73.0 (d)

### (3) Preparation of 5-(2,6-dideoxy-6,6,6-trifluoro-β-L lyxo-hexopyranosyloxy)-6-hydroxynaphtho[2,3-f]quinoline-7,12-dione of the following formula (Ib¹-2) (Compound M of this invention)

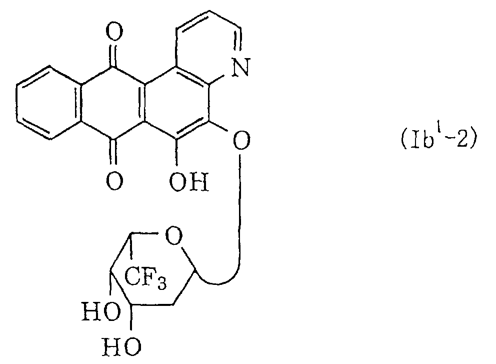

Compound 8b as obtained in Example 4-(1), namely 5-(3,4-di-O-acetyl-2,6-dideoxy-6,6,6-trifluoro-β-L-lyxo-hexopyranosyloxy)-6-hydroxynaphtho[2,3-f]quinoline-7,12-dione (20 mg), was dissolved in a mixture of tetrahydrofuran (2.2 ml) and methanol (1.1 ml). To the resulting solution was added 0.5 M sodium methoxide-methanol solution (0.05 ml). The resulting mixture was allowed to stand for 16 hours, for effecting the deprotection reaction to eliminate the acetyl group from Compound 8b.

Dry ice was added to the resulting reaction solution and the mixture so obtained was concentrated under a reduced pressure. The resulting residue was dissolved in a mixture of chloroform-methanol (5:1). The resulting solution was washed with water, and the remaining organic layer was concentrated under a reduced pressure. The resulting residue was dissolved in a small volume of a mixture of chloroform-methanol (3:1). To the solution as obtained was added isopropylether to cause reprecipitation of the target product, whereby the titled Compound M of this invention was obtained as red solid(14 mg, yield 82 %).
¹H-NMR spectrum (in deuteropyridine)
   δ 5.58(1H, dd, H-1')
   7.38 (1H, dd, H-2)
   9.05 (1H, br d, H-3)
   9.91 (1H, br d, H-1)
¹⁹F-NMR spectrum (in deuteropyridine, CFCl₃ as internal standard)
   δ-72.5 (d)

### Example 5

### (1) Preparation of 5-(3,4-di-O-acetyl-2,6-dideoxy-2-fluoro-a-L-galactopyranosyloxy)-6-hydroxynaphtho[2,3-f] quinoline-7,12-dione (Compound 10a) and 5-(3,4-di-O-acetyl-2,6-dideoxy-2-fluoro-β-L-galactopyranosyloxy)-6-hydroxynaphtho[2,3-f]quinoline-7,12-dione (Compound 10b)

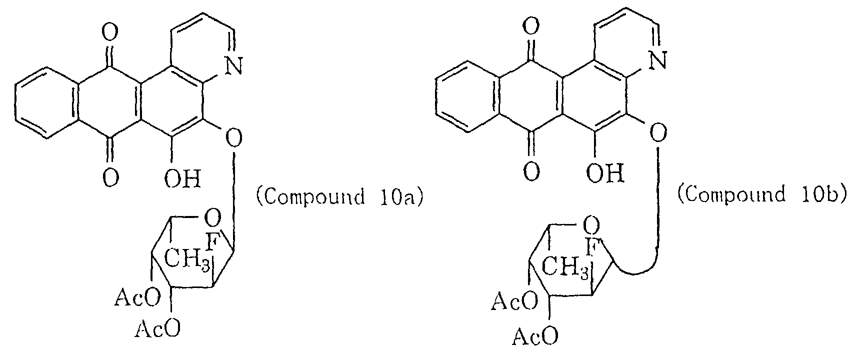

In anhydrous benzene (6.2 ml), there were suspended 3,4-di-O-acetyl-2,6-dideoxy-2-fluoro-α-L-galactopyranosyl bromide (Compound 9) (41 mg) [refer to Y. Takagi, T. Tsuchiya, T. Miyake, T. Takeuchi, and S. Umezawa, the Japanese Journal "Journal of The Society of Organic Synthetic Chemistry", 50, pp.131-142, 1992], Alizarin Blue (54 mg), yellow mercuric oxide (140 mg), mercuric bromide (43 mg) and powdery Molecular Sieves 3A (430 mg). The resulting suspension was refluxed in a dark place under stirring for 2 hours.

The reaction solution so obtained was filtered through Celite to obtain the filtrate. The Celite was washed with benzene. The washings so given were combined with said filtrate, and the combined solution so obtained was washed with a 30 % aqueous potassium iodide solution and then with water. The solution so washed was dried over anhydrous sodium sulfate and then concentrated under a reduced pressure. The resulting residue was subjected to a silica gel column chromatography (developing solvent: chloroform-ethyl acetate, 12:1), for the isolation and purification of the target products. The titled Compound 10a (22 mg, yield 23 %) and the titled Compound 10b (24 mg, yield 25 %) were obtained as red solid, respectively.

### Physical properties of Compound 10a

[α]_{D}²³-106° (c 0.05, chloroform)
¹H-NMR spectrum (in deuterochloroform)
δ 2.11 (3H, s, OAc)
2,18, 13H, s, OAc)
5.05 (1H, ddd, H-2')
6.66 (1H, d, J_{1',2'} = 4Hz, H-1')
7.50 (1H, dd, H-2)
8.80 (1H, dd, H-3)
9.91 (1H, dd, H-1)
12.93 (1H, s, OH)

¹⁹F-NMR spectrum (in deuterochloroform, CFCl₃ as internal standard)
δ -209.1 (ddd)

| | | | |
|---|---|---|---|
| Elemental analysis (for C₂₇H₂₂FNO₉· 0.8H₂O) | | | |
| Calculated | C, 60.29; | H, 4.42; | N, 2.60% |
| Found | C, 60.23; | H, 4.24; | N, 2.46% |

### Physical properties of Compound 10b

[α]_{D}²² -114° (c 0.03, chloroform)
¹H-NMR spectrum (in deuterochloroform)
δ 2.08 (3H, s, OAc)
2.21.(3H, s, OAc)
5.10 (1H, ddd, H-2')
6.17(1H, dd, J_{1',2'} = 8Hz, J_{1',F} = 4Hz, H-1')
7.51 (1H, dd, H-2)
9.02 (1H, dd, H-3)
9.92 (1H, dd, H-1)
12.95 (1H, s, OH)

¹⁹F-NMR spectrum (in deuterochloroform, CFCl₃ as internal standard)
δ -207.4(ddt)

| | | | |
|---|---|---|---|
| Elemental analysis (for C₂₇H₂₂FNO₉·0.5H₂O) | | | |
| Calculated | C, 60.90; | H, 4.35; | N, 2.63% |
| Found | C, 60.83; | H, 4.23; | N, 2.39% |

### (2) Preparation of 5-(2,6-dideoxy-2-fluoro-α-L-galactopyranosyloxy)-6-hydroxynaphtho[2,3-f]quinoline-7,12-dione of the following formula (Ia¹-4) (Compound D of this invention)

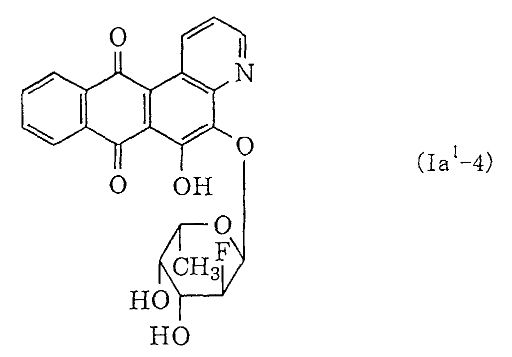

Compound 10a as obtained in Example 5-(1), namely 5- (3, 4-di-O-acetyl-2,6-dideoxy-2-fluoro-α-L-galactopyranosyloxy) -6-hydroxynaphtho[2,3-f]quinoline-7,12-dione (11 mg), was dissolved in a mixture of anhydrous tetrahydrofuran (1 ml) and anhydrous methanol (1 ml). To the resulting solution was added 0.5 M sodium methoxide-methanol solution (3 µl). The resulting mixture was allowed to stand for 15 hours, and was then added with a further mount of 0.5 M sodium methoxide-methanol solution (35 µl). The mixture so obtained was allowed to stand for 16 hours, for effecting the deprotection reaction to eliminate the acetyl group from Compound 10a.

Dry ice was added to the resulting reaction solution, and the mixture so obtained was concentrated under a reduced pressure. The resulting residue was dissolved in chloroform. The resulting solution was washed with water, dried over anhydrous sodium sulfate and then concentrated under a reduced pressure. The resulting residue was reprecipitated from chloroform-hexane, whereby the titled Compound D of this invention was afforded as red solid (8.8 mg, yield 98 %).
[α]_{D}²³ -3° (c 0.03, chloroform)
¹H-NMR spectrum (in deuterochloroform)
δ 4.95 (1H, ddd, H-2')
6.62 (1H, d, H-1')
7.50 (1H, dd, H-2)
8.97 (1H, dd, H-3)
9.91 (1H, dd, H-1)
12.95 (1H, s, OH)

¹⁹F-NMR spectrum (in deuterochloroform, CFCl₃ as internal standard)
δ -211.9 (ddd)

| | | | |
|---|---|---|---|
| Elemental analysis (for C₂₃H₁₈FNO₇ · H₂O) | | | |
| Calculated | C, 60.39; | H, 4.41; | N, 3.06% |
| Found | C, 60.53; | H, 4.14; | N, 2.82% |

### (3) Preparation of 5-(2,6-dideoxy-2-fluoro-β-L-galactopyranosyloxy)-6-hydroxynaphtho[2,3-f]quinoline-7,12-dione of the following formula (Ia¹-5) (Compound E of this invention)

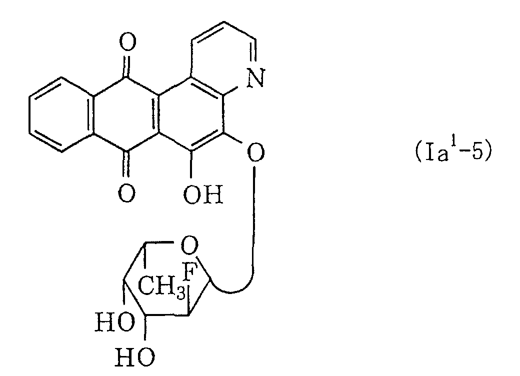

Compound 10b as obtained in Example 5-(1), namely 5-(3, 4-di-O-acetyl-2, 6-dideoxy-2-fluoro-β-L-galactopyranosyloxy) -6-hydroxynaphtho[2,3-f]quinoline-7,12-dione (26 mg), was dissolved in a mixture of anhydrous tetrahydrofuran (2.6 ml) and anhydrous methanol (2.6 ml). To the resulting solution was added a 0.5 M sodium methoxide solution in methanol (85 µl). The resulting mixture was allowed to stand at room temperature for 15 hours, for effecting the deprotection reaction to eliminate the acetyl group from Compound 10b.

Dry ice was added to the resulting reaction solution, and the mixture so obtained was concentrated under a reduced pressure. The resulting residue was dissolved in chloroform. The resulting chloroform solution was washed with water, dried over anhydrous sodium sulfate and then concentrated under a reduced pressure. The residue so obtained was reprecipitated from chloroform-hexane, whereby the titled Compound E of this invention was afforded as red solid(21 mg, yield 96 %).
[α]_{D}²⁴ +182° (c 0.05, chloroform)
¹H-NMR spectrum (in deuterochloroform)
δ 5.51 (1H, dt, H-2')
6.12 (1H, t, H-1')
7.55 (1H, dd, H-2)
8.97 (1H, dd, H-3)
9.96 (1H, dd, H-1)
12.88 (1H, s, OH)

¹⁹F-NMR spectrum (in deuterochloroform, CFCl₃ as internal standard)
δ -190.5 (br dd)

| | | | |
|---|---|---|---|
| Elemental analysis (for C₂₃H₁₈FNO₇ · H₂O) | | | |
| Calculated | C, 60.39; | H, 4.41; | N, 3.06% |
| Found | C, 60.15; | H, 4.34; | N, 2.92% |

### Example 6

### (1) Preparation of 5-[3-O-acetyl-2,4,6-trideoxy-2-fluoro-4-(trifluoroacetamido)-α-L-mannopyranosyloxy]-6-hydroxynaphtho[2,3-f]quinoline-7,12-dione (Compound 12)

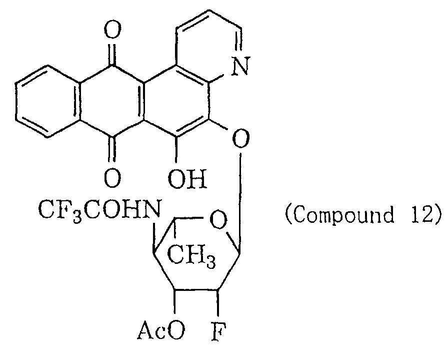

In anhydrous benzene (15.8 ml), there were suspended 3-O-acetyl-2,4,6-trideoxy-2-fluoro-4-(trifluoroacetamido) -α-L-mannopyranosyl bromide (Compound 11) (24.2 mg) (refer to Japanese Patent Application Kokai Hei-9-12590 and European Patent Application First Publication 0834517A1), Alizarin Blue (105 mg), yellow mercuric oxide (353 mg), mercuric bromide (104 mg) and powdery Molecular Sieves 3A (1.27 g). The resulting suspension was refluxed in a dark place under stirring for 13.5 hours.

The reaction solution so obtained was filtered through a layer of Celite to obtain the filtrate. The Celite was washed with benzene. The washings so given were combined with said filtrate, and the combined solution so obtained was washed with a 30 % aqueous potassium iodide solution and then with water. The solution so washed was dried over anhydrous sodium sulfate and then concentrated under a reduced pressure. The resulting residue was subjected to a silica gel column chromatography (developing solvent: chloroform-ethyl acetate, 10:1) and a flash silica gel column chromatography (developing solvent: toluene-ethyl acetate, 6:1), for the isolation and purification of the intended product. The titled Compound 12 of this invention was obtained as reddish brown solid (71 mg, yield 41 %).
[α]_{D}²³ -13° (c 0.1, chloroform)
¹H-NMR spectrum (in deuterochloroform)
δ 2.22 (3H, s, OAc)
5.30 (1H, dt, H-2')
6.17 (1H, dd, J_{1',2'} = 2, J_{1',F} = 6.5Hz, H-1')
7.34 (1H, dd, H-2)
8.72 (1H, dd, H-3)
9.75 (1H, dd, H-1)
12.77 (1H, s, OH)

¹⁹F-NMR spectrum (in deuterochloroform, CFCl₃ as internal standard)
δ -76.5 (3 F, s, CF₃)
-203.7 (1 F,ddd, F-2)

| | | | |
|---|---|---|---|
| Elemental analysis (for C₂₇H₂₀F₄N₂O₈·0.5H₂O) | | | |
| Calculated | C, 55.39; | H, 3.61; | N, 4.78% |
| Found | C, 55.18; | H, 3.48; | N, 4.96% |

### (2) Preparation of 5-(4-amino-2,4,6-trideoxy-2-fluoro-α-L-mannopyranosyloxy)-6-hydroxynaphtho[2,3-f]quinoline-7,12-dione of the following formula (Ia²-1) (Compound F of this invention)

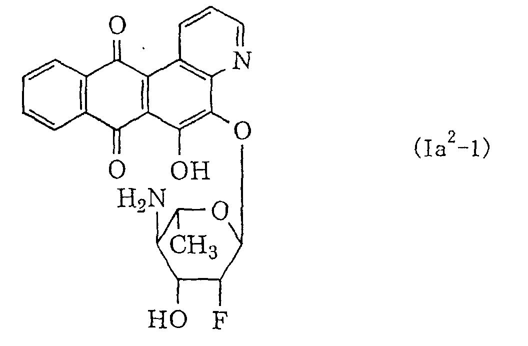

To Compound 12 as obtained in Example 6-(1), namely 5-[3-O-acetyl-2,4,6-trideoxy-2-fluoro-4-(trifluoroacetamido) -α-L-mannopyranosyloxy]-6-hydroxynaphtho[2,3-f]quinoline-7,12-dione (32 mg), was added an aqueous 1M sodium hydroxide solution (3.2 ml). The resulting solution was stirred under an argon atmosphere at 0 °C for 2 hours, for effecting the deprotection reaction to eliminate the trifluoroacetyl group and acetyl group from Compound 12.

The resulting reaction solution was added with 1M hydrochloric acid (3.2 ml) and then added with a saturated aqueous sodium hydrogen carbonate solution (10 ml), and the resultant mixture was extracted with chloroform. The resulting chloroform solution was washed with water, dried over anhydrous sodium sulfate and then concentrated under a reduced pressure. The resulting residue was dissolved in a mixture of chloroform-methanol (2:1), and to the resultant solution was added a 0.54 M hydrogen chloride-methanol solution (0.5 ml). Reprecipitation of the target product was effected by adding isopropylether and hexane to the solution obtained as above. Thus, the titled Compound F of this invention was obtained in the form of its hydrochloride as red solid (20 mg, yield 76 %).
[α]_{D}²² -44° (c 0.1, methanol)
¹H-NMR spectrum (in deuteromethanol)
δ 5.28 (1H, dt, H-2')
6.11 (1H, dd, J_{1',2'} = 2, J_{1',F} = 7Hz, H-1')
7.60 (1H, dd, H-2)
8.98 (1H, dd, H-3)
9.93 (1H, dd, H-1)

¹⁹F-NMR spectrum (in deuteromethanol, CFCl₃ as internal standard)
δ -206.0 (ddd)

| | | | |
|---|---|---|---|
| Elemental analysis (for C₂₃H₁₉FN₂O₆ · HCl ·1.5H₂O) | | | |
| Calculated | C, 55.04; | H, 4.62; | N, 5.58% |
| Found | C, 55.21; | H, 4.49; | N, 5.55% |

### Example 7

### (1) Preparation of 5-[3-O-acetyl-2,4,6-trideoxy-2-fluoro-4-(trifluoroacetamido)-α-L-talopyranosyloxy]-6-hydroxynaphtho[2,3-f]quinoline-7,12-dione (Compound 14)

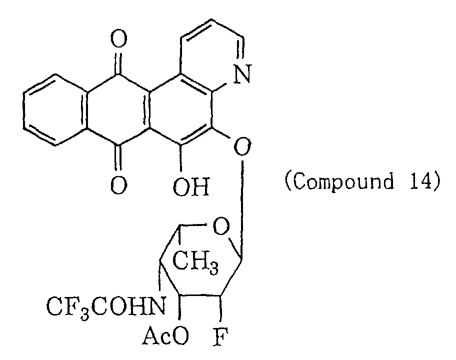

In anhydrous benzene (9.2 ml), there were suspended 3-O-acetyl-2,4,6-trideoxy-2-fluoro-4-(trifluoroacetamido) -α-L-talopyranosyl bromide(Compound 13) (92 mg) [refer to European Patent Application First Publication 0848010A1], Alizarin Blue (61 mg), yellow mercuric oxide (204 mg), mercuric bromide (60 mg) and powdery Molecular Sieves 3A (600 mg) . The resulting suspension was refluxed in a dark place for 15 hours under stirring.

The reaction solution so obtained was filtered through a layer of Celite to obtain the filtrate. The Celite was washed with benzene. The washings so given were combined with said filtrate, and the combined solution was washed with a 30 % aqueous potassium iodide solution and then with water. The solution so washed was dried over anhydrous sodium sulfate and then concentrated under a reduced pressure. The resulting residue was subjected to a flash silica gel column chromatography (developing solvent: toluene-ethyl acetate, 8:1), for the isolation and purification of the intended product. The titled Compound 14 of this invention was obtained as red solid (43.5 mg, yield 36 %).
¹H-NMR spectrum (in deuterochloroform)
   δ 2.15 (3H, s, OAc)
   5.18 (1H, dt, H-2')
   6.06 (1H, dd, J_{1',2'} = 1.5, J_{1',F} = 7.5Hz, H-1')
   7.32 (1H, dd, H-2)
   8.75 (1H, dd, H-3)
   9.78 (1H, dd, H-1)
   12.83 (1H, s, OH)
¹⁹F-NMR spectrum (in deuterochloroform, CFCl₃ as internal standard)
   δ -76.4 (3 F, s, CF₃)
   -199.7 (1 F, ddd, F-2)

### (2) Preparation of 5-(4-amino-2,4,6-trideoxy-2-fluoro-α-L-talopyranosyloxy)-6-hydroxynaphtho[2,3-f]quinoline-7,12-dione of the following formula (Ia²-2) (Compound G of this invention)

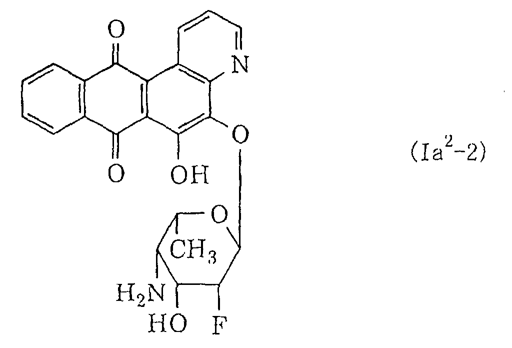

To Compound 14 as obtained in Example 7-(1), namely 5-[3-O-acetyl-2,4,6-trideoxy-2-fluoro-4-(trifluoroacetamido)-α-L-talopyranosyloxy]-6-hydroxynaphtho[2,3-f]quinoline-7,12-dione (28 mg), was added an aqueous 1M sodium hydroxide solution (2.8 ml). The resulting mixture was stirred under an argon atmosphere at 0 °C for 2 hours, for effecting the deprotection reaction to eliminate the trifluoroacetyl group and the acetyl group from Compound 14.

The resulting reaction solution was added with 1M hydrochloric acid (2.8 ml) and then with saturated aqueous sodium hydrogen carbonate solution (8 ml), and the mixture so obtained was extracted with chloroform. The resulting chloroform solution was washed with water, dried over anhydrous sodium sulfate and concentrated under a reduced pressure. The resulting residue was dissolved in a mixture of chloroform-methanol (2:1), and to the resultant solution was added 0.54 M hydrogen chloride-methanol solution (0.45 ml). Reprecipitation of the target product was effected by adding isopropylether and hexane to the solution obtained as above. Thus, the titled Compound G of this invention was obtained in the form of its hydrochloride as red solid (16.6 mg, yield 72 %).
¹H-NMR spectrum (in deuteromethanol)
   δ 5.26 (1H, dt, H-2')
   6.16 (1H, dd, J_{1',2'} = 1.5, J_{1',F} = 7.5Hz, H-1')
   7.62 (1H, dd, H-2)
   8.97 (1H, dd, H-3)
   9.95 (1H, dd, H-1)
¹⁹F-NMR spectrum (in deuteromethanol, CFCl₃ as internal standard)
   δ -202.3 (ddd)

### Example 8

### (1) Preparation of 5-[4-O-acetyl-2,3,6-trideoxy-2-fluoro-3-(trifluoroacetamido)-α-L-talopyranosyloxy]-6-hydroxynaphtho[2,3-f]quinoline-7,12-dione (Compound 16)

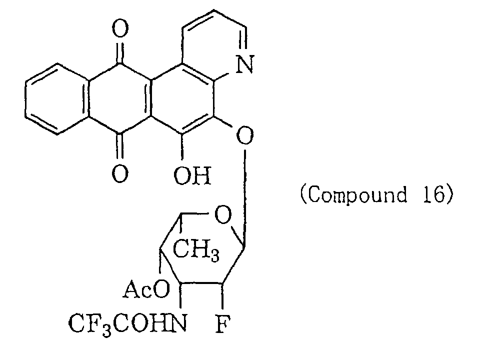

In anhydrous benzene (7.2 ml), there were suspended 4-O-acetyl-2,3,6-trideoxy-2-fluoro-3-(trifluoroacetamido)-α-L-talopyranosyl bromide(Compound 15) (73 mg) [refer to Japanese Patent Application Kokai Sho-64-203397], Alizarin Blue (48 mg), yellow mercuric oxide (162 mg), mercuric bromide (48 mg) and powdery Molecular Sieves 3A (485 mg). The resulting suspension was refluxed in a dark place for 18 hours under stirring.

The reaction solution so obtained was filtered through a layer of Celite to obtain the filtrate. The Celite was washed with benzene. The washings so given were combined with said filtrate, and the combined solution as obtained was washed with a 30 % aqueous potassium iodide solution and then with water. The solution so washed was dried over anhydrous sodium sulfate and then concentrated under a reduced pressure. The resulting residue was subjected to a flash silica gel column chromatography (developing solvent: toluene-ethyl acetate, 7:1), for the isolation and purification of the intended product. The titled Compound 16 of this invention was obtained as red solid (30 mg, yield 32 %).
¹H-NMR spectrum (in deuterochloroform)
   δ 2.18 (3H, s, OAc)
   4.98 (1H, dt, H-2')
   5.93 (1H, dd, J_{1',2'} = 2, J_{1',F} = 8Hz, H-1')
   7.36 (1H, dd, H-2)
   8.78 (1H, dd, H-3)
   9.80 (1H, dd, H-1)
   12.91 (1H, s, OH)
¹⁹F-NMR spectrum (in deuterochloroform, CFCl₃ as internal standard)
   δ -76.8 (3 F, s, CF₃)
   -200.3 (1 F, ddd, F-2)

### (2) Preparation of 5-(3-amino-2,3,6-trideoxy-2-fluoro-α-L-talopyranosyloxy)-6-hydroxynaphtho[2,3-f]quinoline-7,12-dione of the following formula (Ia³-1) (Compound H of this invention)

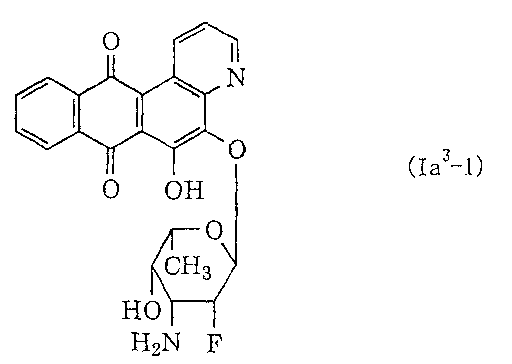

To Compound 16 as obtained in Example 8-(1), namely 5-[4-O-acetyl-2,3,6-trideoxy-2-fluoro-3-(trifluoroacetamido)-α-L-talopyranosyloxy]-6-hydroxynaphtho[2,3-f]quinoline-7,12-dione (22 mg), was added an aqueous 1M sodium hydroxide solution (2.2 ml). The resulting mixture was stirred under an argon atmosphere at 0 °C for 3 hours, for effecting the deprotection reaction to eliminate the trifluoroacetyl group and the acetyl group from Compound 16.

The resulting reaction solution was added with 1M hydrochloric acid (2.2 ml) and then with a saturated aqueous sodium hydrogen carbonate solution (7 ml), and the resultant mixture was extracted with chloroform. The resulting chloroform solution was washed with water, dried over anhydrous sodium sulfate and concentrated under a reduced pressure. The resulting residue was dissolved in a mixture of chloroform-methanol (2:1), and to the resultant solution was added 0.54 M hydrogen chloride-methanol solution (0.35 ml). Reprecipitation of the target product was effected by adding isopropylether and hexane to the solution obtained as above. Thus, the titled Compound H of this invention was afforded in the form of its hydrochloride as red solid (12.5 mg, yield 69 %).
¹H-NNR spectrum (in deuteromethanol)
   δ 4.97 (1H, dt, H-2')
   6.05 (1H, dd, J_{1',2'} = 1.5, J_{1',F} =8Hz, H-1')
   7.63 (1H, dd, H-2)
   8.96 (1H, dd, H-3)
   9.93 (1H, dd, H-1)
¹⁹F-NMR spectrum (in deuteromethanol, CFCl₃ as internal standard)
   δ -199.8 (ddd)

### Example 9

### (1) Preparation of 5-{3-O-[N-(benzyloxycarbonyl)-L-alanyl]-2,6-dideoxy-2-fluoro-α-L-talopyranosyloxy}-6-hydroxynaphtho[2,3-f]quinoline-7,12-dione (Compound 17) and 5-{4-O-[N-(benzyloxycarbonyl)-L-alanyl]-2,6-dideoxy-2-fluoro-α-L-talopyranosyloxy}-6-hydroxynaphtho[2, 3-f]quinoline-7,12-dione (Compound 18)

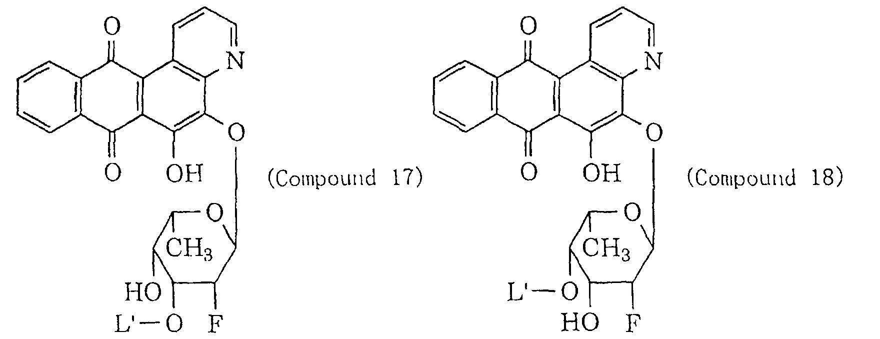

wherein L' represents N-(benzyloxycarbonyl)-L-alanyl group.

Compound A of this invention as obtained in Example 1- (2) (41 mg) was dissolved in anhydrous pyridine (0.5 ml), and the resultant solution was added with N-[N-(benzyloxycarbonyl) -L-alanyloxy]succinimide (75 mg). The resulting mixture was subjected to the reaction at 50 °C for 5 hours.

The reaction solution so obtained was concentrated under a reduced pressure, and the resulting residue was purified by a flash silica gel column chromatography (developer system: chloroform-ethyl acetate, 5:1), thereby affording a mixture of Compound 17 with Compound 18 as a red solid (49 mg, yield, 81 %). The proportion of Compound 17 to Compound 18 in said mixture was about 3:7, as measured by ¹⁹F-NMR spectroscopic analysis, to show that the main product was Compound 18.
¹H-NMR spectrum (in deuterochloroform)
   δ 1.10 (2.1H, d, 5'-Me of Compound 18)
   1.48 (2.1H, d, Me of alanyl group of Compound 18)
   5.13 (1H, br d, H-2')
   6.22 (0.3H, br d, H-1' of Compound 17)
   6.29 (0.7H, br d, H-1' of Compound 18)
   7.28-7.36 (5H, m, Ph)
   7.40 (1H, dd, H-2)
   8.87 (1H, dd, H-3)
   9.75 (1H, dd, H-1)
   12.7 (1H, s, OH)
¹⁹F-NMR spectrum (in deuterochloroform, CFCl₃ as internal standard)
   δ -205.0 (0.7 F, ddd)
   -199.2 (0.3 F, ddd)

### (2) Preparation of 5-(3-O-L-alanyl-2,6-dideoxy-2-fluoro -á-L-talopyranosyloxy)-6-hydroxynaphtho[2,3-f]quinoline-7 ,12-dione of the following formula (Ia⁴-1) (Compound I of this invention), and 5-(4-O-L-alanyl-2,6-dideoxy-2-fluoro-á-L-talopyranosyloxy)-6-hydroxynaphtho[2,3-f]quinoline-7, 12-dione of the following formula (Ia⁴-2) (Compound J of this invention)

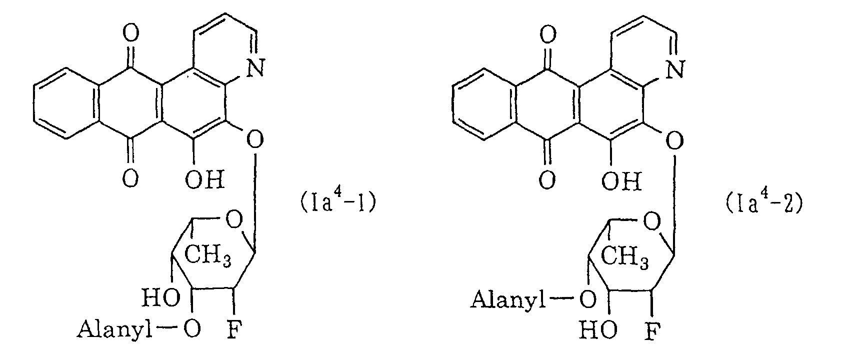

wherein Alanyl represents L-alanyl group.

There was dissolved in acetic acid (1.5 ml) such a mixture (44 mg) of Compound 17 and Compound 18 which were mixed in about 3:7 proportion and which were obtained in the above step (1). To the resultant solution were added 10 % palladium-carbon (44 mg) and 1,4-cyclohexadiene (0.32 ml) which acts as a hydrogen source. The liquid mixture so obtained was stirred at 35 °C under a nitrogen atmosphere for 4 hours. Thus, hydrogenolysis reaction was effected to eliminate the benzyloxycarbonyl group from Compounds 17 and 18.

The resulting reaction solution was filtered through a layer of Celite to obtain the filtrate. The Celite was washed with acetic acid. The filtrate and the resulting acetic acid washings were combined together, and the resultant mixture was added with water (100 ml). The resulting aqueous mixture was washed with chloroform. The aqueous layer as separated from chloroform was concentrated under a reduced pressure and then dried under a reduced pressure, and the resulting residue was suspended in water, which was then extracted with chloroform. The resulting chloroform solution was concentrated under a reduced pressure, to afford a mixture of Compound I and Compound J of this invention as a red solid (7.8 mg). ¹⁹F-NMR spectrum (in deuterochloroform, CFCl₃ as internal standard)
δ -205.0 (0.7 F, ddd)
-201.8 (0.3 F, ddd)
FAB mass spectrum: 511 (M⁺ + 1)

### Example 10

### (1) Preparation of 5-{3,4-di-O-[N-(benzyloxycarbonyl) -L-alanyl]-2,6-dideoxy-2-fluoro-á-L-talopyranosyloxy}-6-h ydroxynaphtho[2,3-f]-quinoline-7,12-dione (Compound 19)

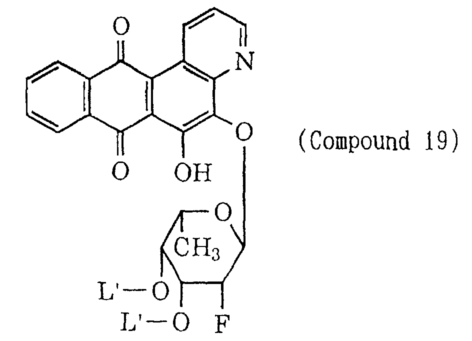

wherein L' represents N-(benzyloxycarbonyl)-L-alanyl group.

Compound A of this invention as obtained in Example 1-(2) (13 mg) was dissolved in anhydrous pyridine (0.2 ml), and the resulting solution was added with N-[N-(benzyloxycarbonyl)-L-alanyloxy]succinimide (54 mg). The resulting reaction mixture was subjected to the reaction at 60 °C for 13.5 hours. There occurred the reaction for esterifying the 3'- and 4'-hydroxyl groups of Compound A of this invention with N-benzyloxycarbonyl-L-alanyl group. The reaction solution so obtained was concentrated under a reduced pressure, and the resulting residue was purified by a flash silica gel column chromatography (developer system: chloroform-ethyl acetate, 5:1), to afford the titled Compound 19 of this invention as a red solid (18 mg, yield, 71 %).
¹H-NMR spectrum (in deuterochloroform)
   δ 1.13 (3H, d, 5'-Me Compound 19)
   1.43 (3H, d, Me of alanyl group)
   1.48 (3H, d, Me of alanyl group)
   5.28 (1H, d, H-2')
   6.34 (1H, d, H-1')
   7.50 (1H, dd, H-2)
   8.96 (1H, dd, H-3)
   9.90 (1H, dd, H-1)
   12.8 (1H, s, OH)
¹⁹F-NMR spectrum (in deuterochloroform, CFCl₃ as internal standard)
   δ -203.0 (m)

### (2) Preparation of 5-(3,4-di-O-L-alanyl-2,6-dideoxy-2-fluoro-α-L-talopyranosyloxy)-6-hydroxynaphtho[2,3-f] quinoline-7,12-dione of the following formula (Ia⁴-3) (Compound K of this invention)

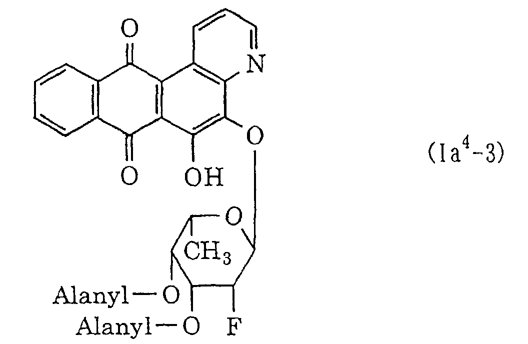

wherein Alanyl represents L-alanyl group.

Compound 19 as obtained in the above step (1) (18 mg) was dissolved in acetic acid (0.4 ml), and to the resulting solution were added 10 % palladium-carbon (18 mg) and 1,4-cyclohexadiene (0.2 ml). The resultant liquid mixture was stirred at 35 °C under a nitrogen atmosphere for 28 hours to effect the hydrogenolysis reaction. During the reaction, there were further added acetic acid (0.6 ml) and 1,4-cyclohexadiene (0.5 ml) to the reaction mixture.

The reaction solution so obtained was filtered through a layer of Celite to obtain the filtrate. The Celite was washed with acetic acid. The filtrate and the resulting acetic acid washings were combined together, and the resulting solution as combined was concentrated under a reduced pressure. The resulting residue was dissolved in a mixture of chloroform (3 ml), methanol (1 ml) and trifluoroacetic acid (0.1 ml) in an ice bath. Isopropylether was added to the solution so obtained, and the resulting red solid as precipitated was recovered by centrifugation. Thus, the titled Compound K of this invention was obtained in the form of a trifluoroacetate (7.9 mg, yield, 55 %).
¹⁹F-NMR spectrum (in deuterochloroform, CFCl₃ as internal standard)
   δ -202.9 (1 F, ddd)
   -75.3 (3 F, s, CF₃COOH)
FAB mass spectrum: 582 (M⁺ + 1)ₒ
   The following Examples 11 - 13 are given to illustrate the preparation of several compounds of the general formula (V), particularly some compounds of the general formula (Va), which are provided according to the third aspect of this invention.

### Example 11

### (1) Preparation of 5-[3-O-(3-tertiary-butoxycarbonylaminopropionyl)-2,6-dideoxy-2-fluoro-α-L-talopyranosyloxy] -6-hydroxynaphtho[2,3-f]quinoline-7,12-dione (Compound N' of this invention), and 5-[4-O-(3-tertiary-butoxycarbonylaminopropionyl)-2,6-dideoxy-2-fluoro-α-L-talopyranosyloxy] -6-hydroxynaphtho[2,3-f]quinoline-7,12-dione (Compound O' of this invention), and 5-[3,4-di-O-(3-tertiary-butoxycarbonylaminopropionyl)-2,6-dideoxy-2-fluoro-α-L-talopyranosyloxy] -6-hydroxynaphtho[2,3-f] quinoline-7,12-dione (Compound P' of this invention)

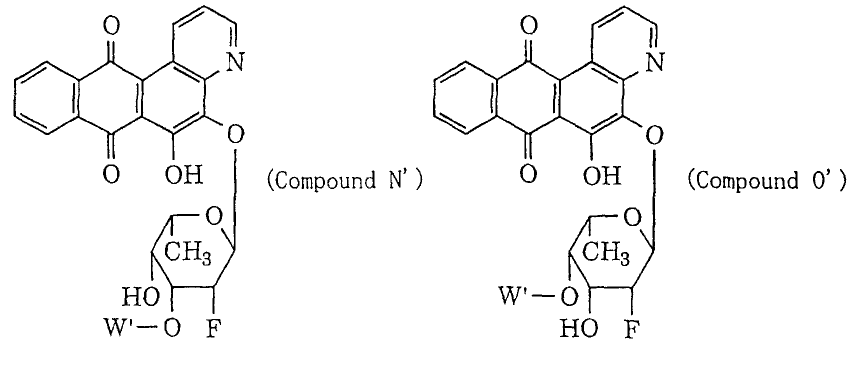

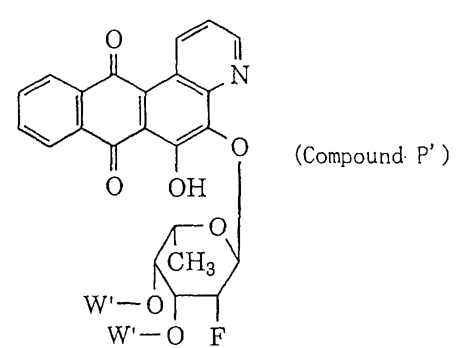

wherein W' represents 3-tertiary-butoxycarbonylaminopropionyl group -CO-CH₂CH₂-NH-CO-OC(CH₃)₃.

Compound of formula (Ia¹-1) as obtained in Example 1-(2), namely 5-(2,6-dideoxy-2-fluoro-α-L-talopyranosyloxy)-6-hydroxynaphtho[2,3-f]quinoline-7,12-dione (Compound A of this invention), (24.6 mg) was dissolved in anhydrous dichloromethane (2.5 ml). To the resulting solution, there were added 3-tertiary-butoxycarbonylaminopropionic acid (30.2 mg), 1-ethyl-3-(3-dimethylaminopropyl)carbodiimide hydrochloride (25.6 mg) and 4-dimethylaminopyridine(3.5 mg). The resulting mixture was stirred at room temperature for 17 hours. The reaction solution thus obtained was diluted with chloroform, and washed successively with 20 % aqueous potassium hydrogen sulfate solution, a saturated aqueous sodium hydrogen carbonate solution and water. The reaction solution so washed was dried over anhydrous sodium sulfate and concentrated under a reduced pressure. The resulting residue was subjected to a silica gel column chromatography (developer system: toluene-ethyl acetate, 2:1) for the separation and purification of the target products. Thus, a mixture of the titled Compounds N' and O' (9.7 mg, yield, 28 %; the proportion of Compounds N' to O': was 3:1), as well as the titled Compound P' (7.7 mg, yield, 18 %), were obtained each in the form of orange colored solid.

### Compound N' and Compound O'

¹H-NMR spectrum (in deuterochloroform)
   δ 1.12, 1.28 (s of 2.3H and 0.7H, respectively, CH₃-5')
   1.43, 1.45 (9H in combination, each s, -C(CH₃)₃)
   3.99 (0.75H, brdd, H-4' of Compound N')
   4.54 (0.25H, ddt, H-3' of Compound O')
   5.58 (0.75H, dt, H-3' of Compound N')
   12.82, 12.83 (1H in combination, each s, OH-6)
¹⁹F-NMR spectrum (in deuterochloroform, CFCl₃ as internal standard)
   δ -199.2 (0.75F, ddt, F-2' of Compound N')
   -204.8 (0.25F, ddd, F-2' of Compound O')

### Compound P'

¹H-NMR spectrum (in deuterochloroform)
   δ 1.16 (3H, d, CH₃-5')
   1.44, 1.46 (18H in combination, each s, -C(CH₃)₃)
   2.64 (4H, m, COCH₂)
   5.47 (1H, br d, H-4')
   5.75 (1H, dt, H-3')
   7.51 (1H, dd, H-2)
   8.96 (1H, dd, H-3)
   9.92 (1H, dd, H-1)
   12.84 (1H, s, OH-6)
¹⁹F-NMR spectrum (in deuterochloroform, CFCl₃ as internal standard)
   δ

### (2) Preparation of 5-[3-O-(3-aminopropionyl)-2,6-dideoxy-2-fluoro-α-L-talopyranosyloxy]-6-hydroxynaphtho [2,3-f]quinoline-7,12-dione (Compound N of this invention), and 5-[4-O-(3-aminopropionyl)-2,6-dideoxy-2-fluoro-α-L-talopyranosyloxy]-6-hydroxynaphtho[2,3-f]quinoline-7,12-dione (Compound O of this invention)

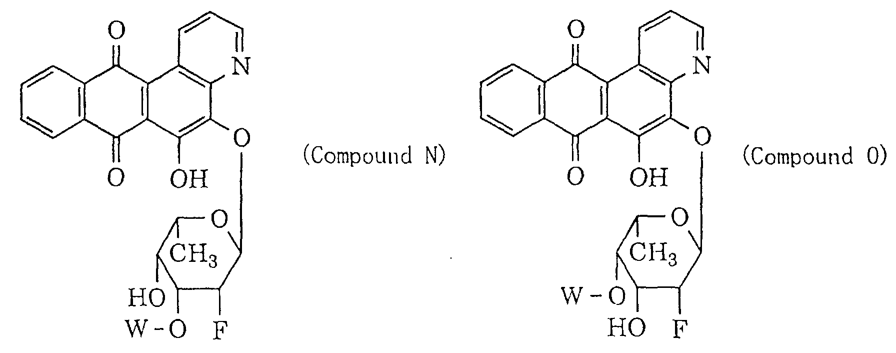

wherein W represents 3-aminopropionyl group -CO-CH₂CH₂-NH₂.

The mixture of Compound N' and Compound O' as obtained in the above step (1) (7.2 mg) was dissolved in trifluoroacetic acid (0.04 ml)at -15 °C. The resultant solution was allowed to stand at -5 °C for 10 minutes, to eliminate the tertiary-butoxycarbonyl group from the said compounds. Isopropylether was added to the resulting reaction solution to cause precipitation. The resulting precipitate was separated and washed with isopropylether, whereby a mixture of the titled Compound N and Compound O was obtained as an orange colored solid in the form of a trifluoroacetate thereof (7 mg, yield, 95 %; the proportion of Compound N to Compound O was 3:2)

### Compound N and Compound O

¹H-NMR spectrum (in deuteromethanol)
   δ 1.08 (1.2H, d, CH₃-5' of Compound O)
   1.20 (1.8H, d, CH₃-5' of Compound N)
   2.95 (2H, m, COCH₂)
   4.55 (0.4H, br dt, H-3' of Compound O)
   5.54 (0.6H, br dt, H-3' of Compound N)
¹⁹F-NMR spectrum (in deuteromethanol, CFCl₃ as internal standard)
   δ -202.6 (0.4F, ddd, F-2' of Compound O)
   -200.0 (0.6F, ddd, F-2' of Compound N)
   -75.5 (3F, s, CF₃COOH)

### (3) Preparation of 5-[3,4-di-O-(3-aminopropionyl)-2,6-dideoxy-2-fluoro-α-L-talopyranosyloxy]-6-hydroxynaphtho [2,3-f]quinoline-7,12-dione (Compound P of this invention)

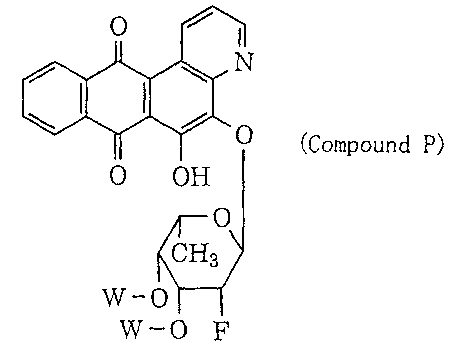

wherein W represents 3-aminopropionyl group.

Compound P' as obtained in the above step (1) (6.9 mg) was dissolved in trifluoroacetic acid (0.03 ml) at -15 °C. The resulting solution was allowed to stand at -5°C for 10 minutes, to eliminate the tertiary-butoxycarbonyl group from the said compound. Isopropylether was added to the resulting reaction solution to cause precipitation. The resulting precipitate was separated and washed with isopropylether, whereby the titled Compound P was obtained as reddish orange colored solid in the form of its trifluoroacetate (5.8 mg, yield, 81 %).
¹H-NMR spectrum (in deuteromethanol)
   δ 1.10 (3H, d, CH₃-5')
   2.86 (4H, m, COCH₂)
   5.47 (1H, br d, H-4')
   5.79 (1H, dt, H-3')
   6.28 (1H, br dd, H-1')
   7.59 (1H, dd, H-2)
   8.97 (1H, dd, H-3)
   9.93 (1H, dd, H-1)
¹⁹F-NMR spectrum (in deuteromethanol, CFCl₃ as internal standard)
   δ -201.1 (1F, ddd, F-2')
   -75.5 (6F, s, CF₃COOH)

### Example 12

### (1) Preparation of 5-[3,4-di-O-(3-tertiary-butoxycarbonylaminopropionyl)-2,6-dideoxy-2-fluoro-α-L-galactopyranosyloxy] -6-hydroxynaphtho[2,3-f]quinoline-7,12-dione (Compound Q' of this invention)

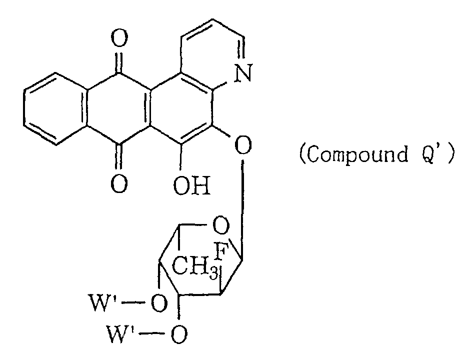

wherein W' represents 3-tertiary-butoxycarbonylaminopropionyl group.

The compound of formula (Ia¹-4) as obtained in Example 5-(2) above, namely 5-(2,6-dideoxy-2-fluoro-α-L-galactopyranosyloxy)-6-hydroxynaphtho[2,3-f]quinoline-7,12-dione (Compound D of this invention), (20mg) was dissolved in anhydrous dichloromethane (2 ml). To the resulting solution, there were added 3-tertiary-butoxycarbonylaminopropionic acid (24.5 mg), 1-ethyl-3-(3-dimethylaminopropyl) carbodiimide hydrochloride(22 mg) and 4-dimethylaminopyridine (14.5 mg). The resulting mixture was stirred at room temperature for 2.5 hours. Then, there were further added to said mixture 3-tertiary-butoxycarbonylaminopropionic acid (4 mg) and 1-ethyl-3-(3-dimethylaminopropyl)carbodiimide hydrochloride (4 mg). The resultant mixture was stirred at room temperature for further 16 hours. The reaction solution thus obtained was diluted with chloroform, and washed successively with a 20 % aqueous potassium hydrogen sulfate solution, a saturated aqueous sodium hydrogen carbonate solution and water. The reaction solution so washed was dried over anhydrous sodium sulfate and concentrated under a reduced pressure. The resulting residue was subjected to a silica gel column chromatography (developer system: toluene-ethyl acetate, 2:1), for the isolation and purification of the final product. Thus, the titled Compound Q' was obtated as red solid (28 mg, yield, 80 %).
¹H-NMR spectrum (in deuterochloroform)
δ 1.44, 1.46 (each 9H, s, -C(CH₃)₃)
5.03 (2H, ddd, H-2')
5.60 (1H, br ddd, H-4')
6.02 (1H, ddd, H-3')
6.67 (1H, d, H-1')
7.51 (1H, dd, H-2)
8.98 (1H, dd, H-3)
9.92 (1H, dd, H-1)
12.95 (1H, s, OH-6)

¹⁹F-NMR spectrum (in deuterochloroform, CFCl₃ as internal standard)
δ -208.9 (ddd)

| | | | |
|---|---|---|---|
| Elemental analysis Elemental analysis (for C₃₉H₄₄FN₃O₁₃ · H₂O) | | | |
| Calculated | C, 58.87; | H, 5.79; | N, 5.25% |
| Found | C, 58.87; | H, 5.52; | N, 5.29% |

### (2) Preparation of 5-[3,4-di-O-(3-aminopropionyl)-2,6-dideoxy-2-fluoro-α-L-galactopyranosyloxy]-6-hydroxynaphtho [2,3-f]quinoline-7,12-dione (Compound Q of this invention)

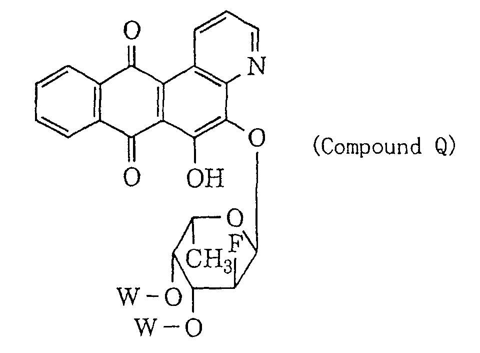

wherein W represents 3-aminopropionyl group.

Compound Q' as obtained in the above step (1) (61 mg) was dissolved in trifluoroacetic acid (0.3 ml) at 0 °C. The resulting solution was allowed to stand at 0 °C for 30 minutes to eliminate the tertiary-butoxycarbonyl group from the compound Q'. Isopropylether was added to the resulting reaction solution to cause precipitation. The precipitate so formed was separated and washed with isopropylether, to afford the titled Compound Q in the form of a trifluoroacetate thereof, as reddish brown colored solid (54 mg, yield, 86 %).
¹H-NMR spectrum (in deuteromethanol)
δ 1.04 (3H, d, CH₃-5')
2.75-2.98 (4H, m, COCH₂×2)
3.27 (4H, m, CH₂NH₂×2)
6.68 (1H, d, H-1')
7.54 (1H, dd, H-2)
8.94 (1H, dd, H-3)
9.84 (1H, dd, H-1)

¹⁹F-NMR spectrum (in deuteromethanol, CFCl₃ as internal standard)
δ -208.1 (1F, ddd, F-2')
-75.3 (6F, s, CF₃COOH)

| | | | |
|---|---|---|---|
| Elemental analysis (for C₃₃H₃₀F₇N₃O₁₃ · 2.5H₂O) | | | |
| Calculated | C, 46.38; | H, 4.13; | N, 4.92% |
| Found | C, 46.23; | H, 4.03; | N, 4.89% |

### Example 13

### (1) Preparation of 5-[3-O-(3-tertiary-butoxycarbonylaminopropionyl) -2,6-dideoxy-2-fluoro-β-L-galactopyranosyloxy] -6-hydroxynaphtho[2,3-f]quinoline-7,12-dione (Compound R' of this invention), and 5-[3,4-di-O-(3-tertiary-butoxycarbonylaminopropionyl)-2,6-dideoxy-2-fluoro-β-L-galactopyranosyloxy]-6-hydroxynaphtho[2,3-f]quinoline-7,12-dione(Compound S' of this invention), and 5-[3,4-di-O-(3-tertiary-butoxycarbonylaminopropionyl)-2,6-dideoxy-2-fluoro-β-L-galactopyranosyloxy]-6-(3-tertiary-butoxycarbonylaminopropionyloxy)-naphtho[2,3-f]quinoline-7,12-dione (Compound T' of this invention)

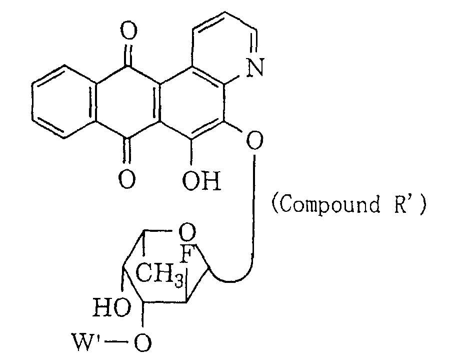

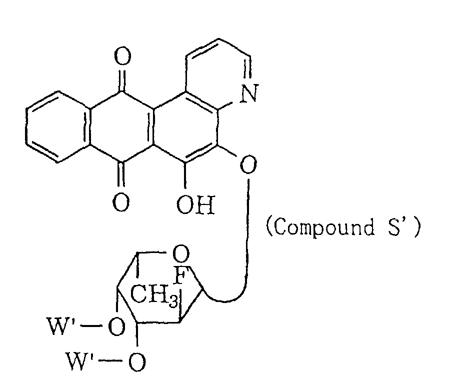

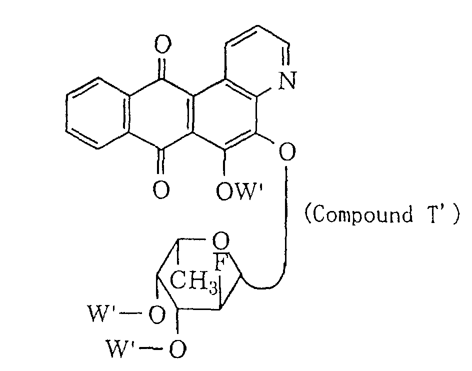

wherein W' represents3-tertiary-butoxycarbonylaminopropionyl group.

The compound of formula (Ia¹-5) as obtained in Example 5-(3) above, namely 5-(2,6-dideoxy-2-fluoro-β-Lgalactopyranosyloxy)-6-hydroxynaphtho[2,3-f]quinolone-7, 12-dione (Compound E of this invention) (23, 8 mg) was dissolved in anhydrous dichloromethane (2.4 ml). To the resulting solution, there were added 3-tertiary-butoxycarbonylaminopropionic acid (24.7 mg), 1-ethyl-3-(3-dimethylaminopropyl)carbodiimide hydrochloride (26.9 mg) and 4-dimethylaminopyridine(4.3 mg). The resulting mixture was stirred at room temperature for 16 hours. The reaction solution thus obtained was diluted with chloroform, and washed successively with a 20 % aqueous potassium hydrogen sulfate solution, a saturated aqueous sodium hydrogen carbonate solution and water. The reaction solution so washed was then dried over anhydrous sodium sulfate and concentrated under a reduced pressure. The resulting residue was subjected to a silica gel column chromatography (developer system: toluene-ethyl acetate, 2:1), for the isolation and purification of the target products. Thus, the titled Compound R' (6.6 mg, yield, 20 %) and Compound S' (14.3 mg, yield, 34 %), respectively, were obtained as red solid. Further, the titled Compound T' (8.5 mg, yield, 16,5 %) was obtained as yellow solid.

### Compound R'

¹H-NMR spectrum (in deuterochloroform)
   δ 1.41 (9H, s, -C(CH₃)₃)
   3.97 (1H, br ddd, H-4')
   5.13 (1H, m, H-3')
   7.52 (1H, dd, H-2)
   8.98 (1H, dd, H-3)
   9.94 (1H, dd, H-1)
   12.94 (1H, s, OH-6)
¹⁹F-NMR spectrum (in deuterochloroform, CFCl₃ as internal standard)
   δ -203.7 (br ddd)

### Compound S'

¹H-NMR spectrum (in deuterochloroform)
   δ 1.45 (18H, s, -C(CH₃)₃)
   5.26 (1H, m, H-3')
   5.35 (1H, br d, H-4')
   7.51 (1H, dd, H-2)
   9.01 (1H, dd, H-3)
   9.91 (1H, dd, H-1)
   12.93 (1H, s, OH-6)
¹⁹F-NMR spectrum (in deuterochloroform, CFCl₃ as internal standard)
   δ -207.1 (brdd)

### Compound T'

[α]_{D}²¹ -100° (c 0.05, chloroform)
¹H-NMR spectrum (in deuterochloroform)
   δ 1.45, 1.49 (18H and 9H, respectively, s, -c(CH₃)₃)
   5.27 (1H, m, H-3')
   5.68 (1H, br s, H-4')
   7.69 (1H, dd, H-2)
   9.07 (1H, dd, H-3)
   10.01 (1H, dd, H-1)
¹⁹F-NMR spectrum (in deuterochloroform, CFCl₃ as internal standard)
   -207.4 (br d)

### (2) Preparation of 5-[3-O-(3-aminopropionyl)-2,6-dideoxy-2-fluoro-β-L-galactopyranosyloxy]-6-hydroxynaphtho [2,3-f]quinoline-7,12-dione (Compound R of this invention)

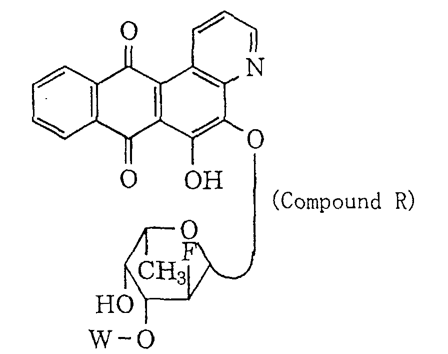

wherein W represents 3-aminopropionyl group.

Compound R' as obtained in the above step (1) (6.4 mg) was dissolved in trifluoroacetic acid (0.03 ml) at 0 °C. The solution was allowed to stand at 0°C for 5 minutes, to eliminate the tertiary-butoxycarbonyl group from the compound R'. Isopropylether was added to the resulting reaction solution to cause precipitation. The resulting precipitate was separated and washed with isopropylether, whereby the titled Compound R was obtained as reddish orange colored solid in the form of its trifluoroacetate (5.8 mg, yield, 89 %).
¹H-NMR spectrum (in deuteromethanol)
   δ 1.09 (3H, d, CH₃-5')
   2.93 (2H, m, COCH₂)
   3.90 (1H, brt, H-4')
   5.23 (1H, ddd, H-3')
   6.08 (1H, dd, H-1')
   7.59 (1H, dd, H-2)
   8.94 (1H, dd, H-3)
   9.89 (1H, br dd, H-1)
¹⁹F-NMR spectrum (in deuteromethanol, CFCl₃ as internal standard)
   δ -207.1 (1F, ddt, F-2')
   -75.3 (3F, s, CF₃COOH)

### (3) Preparation of 5-[3,4-di-O-(3-aminopropionyl)-2,6-dideoxy-2-fluoro-β-L-galactopyranosyloxy)-6-hydroxynaphtho [2,3-f]quinoline-7,12-dione (Compound S of this invention)

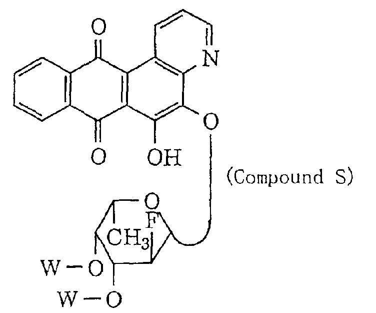

wherein W represents 3-aminopropionyl group.

Compound S' as obtained in Example 13-(1) above (13.3 mg) was dissolved in trifluoroacetic acid (0.07 ml) at 0 °C. The resulting solution was allowed to stand at 0°C for 5 minutes, to eliminate the tertiary-butoxycarbonyl group from the compound S'. Isopropylether was added to the resulting reaction solution to cause precipitation. The resulting precipitate was separated and washed with isopropylether, whereby the titled Compound S was obtained as orange colored solid in the form of its trifluoroacetate (11.4 mg, yield, 83 %).
¹H-NMR spectrum (in deuteromethanol)
δ 1.08 (3H, d, CH₃-5')
2.82 (2H, m, COCH₂)
2.97 (2H, m, COCH₂)
5.38 (1H, br s, H-4')
5.51 (1H, ddd, H-3')
6.27 (1H, dd, H-1')
7.59 (1H, dd, H-2)
8.94 (1H, dd, H-3)
9.88 (1H, dd, H-1)

¹⁹F-NMR spectrum (in deuteromethanol, CFCl₃ as internal standard)
δ -206.8 (1F, ddd, F-2')
-75.5 (6F, s, CF₃COOH)

| | | | |
|---|---|---|---|
| Elemental analysis (for C₃₃H₃₀F₇N₃O₁₃ · 2.5H₂O) | | | |
| Calculated | C, 46.38; | H, 4.13; | N, 4.92% |
| Found | C, 46.54; | H, 3.89; | N, 4.86% |

### (4) Preparation of 5-[3,4-di-O-(3-aminopropionyl)-2,6-dideoxy-2-fluoro-β-L-galactopyranosyloxy)-6-(3-aminopropionyloxy)-naphtho[2,3-f]quinoline-7,12-dione (Compound T of this invention)

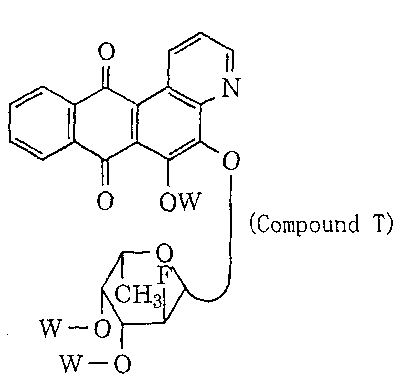

wherein W represents 3-aminopropionyl group.

Compound T' as obtained in Example 13-(1) (8.3 mg) was dissolved in trifluoroacetic acid (0.04 ml) at 0 °C. The resulting solution was allowed to stand at 0 °C for 5 minutes, to eliminate the tertiary-butoxycarbonyl group from the compound T'. Isopropylether was added to the resulting reaction solution to cause precipitation. The resulting precipitate was separated and washed with isopropylether, whereby the titled Compound T was obtained as pale yellow solid in the form of its trifluoroacetate (7.5 mg, yield, 87 %). [α]_{D}²¹ -6° (c 0.05, methanol)
¹H-NMR spectrum (in deuteromethanol)
δ 1.07 (3H, d, CH₃-5')
2.81 (2H, m, COCH₂)
3.01 (2H, m, COCH₂)
5.36 (1H, br s, H-4')
5.50 (1H, ddd, H-3')
7.82 (1H, dd, H-2)
9.08 (1H, dd, H-3)
10.02 (1H, dd, H-1)

¹⁹F-NMR spectrum (in deuteromethanol, CFCl₃ as internal standard)
δ -206.1 (1F, br d, F-2')
-75.0 (9F, s, CF₃COOH)

| | | | |
|---|---|---|---|
| Elemental analysis (for C₃₈H₃₆F₁₀N₄O₁₆ · 2.5H₂O) | | | |
| Calculated | C, 43.89; | H, 3.97; | N, 5.39% |
| Found | C, 43.81; | H, 3.74; | N, 5.47% |

### INDUSTRIAL APPLICABILITY

As explained hereinbefore, according to this invention, there are provided a 5-L or D-glycosyloxy-6-hydroxynaphtho [2,3-f]quinoline-7,12-dione of the general formula (I), particularly a naphtho[2,3-f]quinoline derivative of the general formulae (Ia) and (Ib), as well as a naphtho[2,3-f]-quinoline derivative of the general formula (V), particularly a naphtho[2,3-f]quinoline derivative of the general formula (Va). These compounds of this invention are useful as anticancer agent or antitumor agent.

## Claims

1. A 5-L or D-glycosyloxy-6-hydroxynaphtho[2,3-f] quinoline-7,12-dione represented by the following general formula (I) 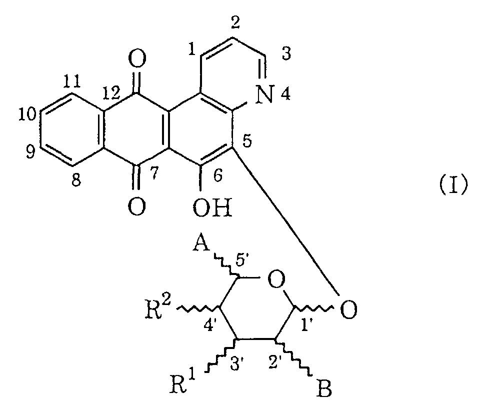 wherein A is methyl group or trifluoromethyl group, B is a hydrogen atom or an electron-withdrawing group chosen from fluoro group, chloro group, bromo group, iodo group, difluoro group (-F₂), an alkoxy group of 1-5 carbon atoms and cyano group (-CN), with provisos that when A is trifluoromethyl group, B stands for hydrogen atom, and that when A is methyl group, B stands for such electron-withdrawing group as above, and R¹ is either hydroxyl group or amino group, or a hydroxyl group having been esterified with an α-amino acid residue represented by the following formula (a) 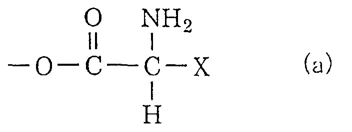 where X is hydrogen atom or such an alkyl group or such a substituted alkyl group which is known to be combined to the α-carbon atom of a known α-amino acid molecule; or R¹ is a hydroxyl group having been esterified with an acyl group represented by the following formula (b) 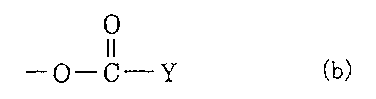 where Y is hydrogen atom or a lower alkyl group, or Y is an aryl group, particularly phenyl group or a substituted phenyl group, or Y is an aralkyl group, particularly benzyl group or a substituted benzyl group; or R¹ is a hydroxyl group having been O-glycosylated with a pentose or a hexose, and R² has the same meaning as defined for R¹, and a pharmaceutically acceptable acid-addition salt thereof.

2. A compound as claimed in Claim 1, wherein the glycosyl group which is present in the compound of general formula (I) and which is represented by the following formula (c) 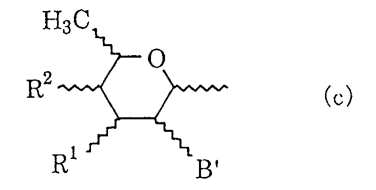 is such an L or D-glycosyl group which has the configuration of 2,6-dideoxy or 6-deoxy-α or β-L or D-talopyranosyl group; or 2,6-dideoxy or 6-deoxy-α or β-L or D-galactopyranosyl group; or 2, 6-dideoxy or 6-deoxy-α or β-L or D-mannopyranosyl group; or 2,6-dideoxy-α or β-L or D-lyxo-hexopyranosyl group, or alternatively wherein the glycosyl group of formula (c) has the configuration of 4-amino-2,4,6-trideoxy- or 3-amino-2,3,6-trideoxy-2-substituted-talopyranosyl or galactopyranosyl or mannopyranosyl group.

3. A compound as claimed in Claim 1, wherein the compound of general formula (I) shown in Claim 1 is a 5-(2,6-dideoxy or 6-deoxy-2-substituted-3-O-substituted or unsubstituted-or 4-O-substituted or unsubstituted- or 3,4-O-di-substituted or unsubstituted-L or D-glycosyloxy)-6-hydroxynaphtho [2,3-f]quinoline-7,12-dione represented by the following general formula (Ia) 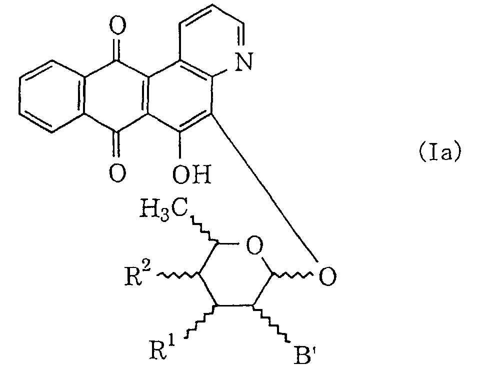 wherein B' is an electron-withdrawing group chosen from fluoro group, chloro group, bromo group, iodo group, difluoro group (-F₂), an alkoxy group of 1-5 carbon atoms and cyano group (-CN), and R¹ is either hydroxyl group or amino group, or a hydroxyl group having been esterified with an α-amino acid residue of the following formula (a) 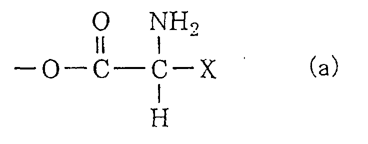 where X is hydrogen atom or such an alkyl group or such a substituted alkyl group which is known to be combined to the α-carbon atom of a known α-amino acid molecule; or R¹ is a hydroxyl group having been esterified with an acyl group of the following formula (b) 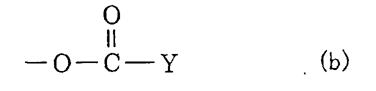 where Y is a lower alkyl group or an aryl group, particularly phenyl group or a substituted phenyl group, or Y is an aralkyl group, particularly benzyl group or a substituted benzyl group; or R¹ is a hydroxyl group having been O-glycosylated with a pentose or a hexose, and R² has the same meaning as defined for R¹.

4. A compound as claimed in Claim 3, wherein the glycosyl group which is present in the compound of general formula (Ia) and which is represented by the following formula (c') 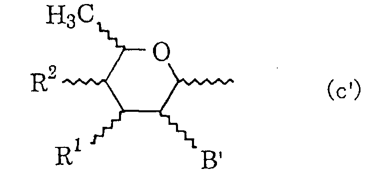 where B', R¹ and R² have the same meanings as defined in Claim 3 is such a glycosyl group which has the configuration of 2,6-dideoxy or 6-deoxy-α or β-L or D-talopyranosyl group; or 2,6-dideoxy or 6-deoxy-α or β-L or D-galactopyranosyl group; or 2,6-dideoxy or 6-deoxy-α or β-L or D-mannopyranosyl group.

5. A compound as claimed in Claim 3, wherein the compound is a compound of general formula (Ia) in which B' is fluoro group, chloro group, bromo group, difluoro group, methoxy group, ethoxy group or cyano group, and R¹ is hydroxyl group, amino group, glycyloxy group, alanyloxy group or acetoxy group, and R² is hydroxyl group, amino group, glycyloxy group, alanyloxy group or acetoxy group.

6. A compound as claimed in Claim 3, wherein the compound of general formula (Ia) is a 5-(2,6-dideoxy or 6-deoxy-2-substituted-L or D-talopyranosyloxy or galactopyranosyloxy or mannopyranosyloxy)-6-hydroxynaphtho[2,3-f]quinoline-7, 12-dione represented by general formula (Ia¹) 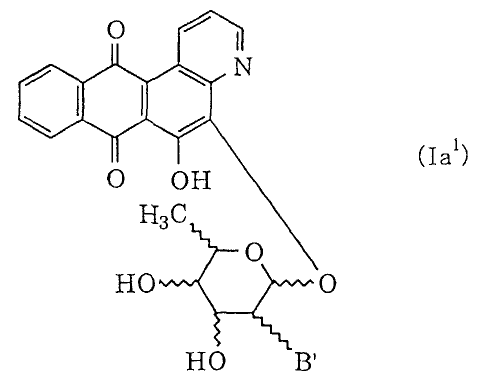 where B' is an electron-withdrawing group having the same meaning as defined in Claim 3.

7. A compound as claimed in Claim 6, wherein the compound of general formula (Ia¹) is 5-(2,6-dideoxy-2-fluoro-α-L-talopyranosyloxy)-6-hydroxynaphtho[2,3-f]quinoline-7,12-dione represented by the following formula (Ia¹-1) 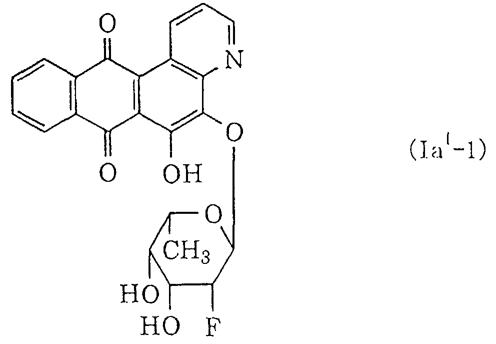 or is 5-(6-deoxy-2-O-methyl-α-L-talopyranosyloxy)-6-hydroxynaphtho[2,3-f]quinoline-7,12-dione represented by the following formula (Ia¹-2) 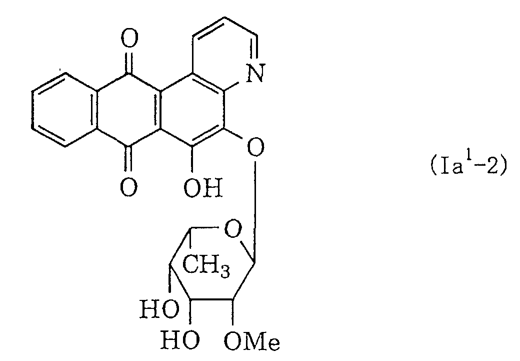 where Me is methyl group.

8. A compound as claimed in Claim 6, wherein the compound of general formula (Ia¹) is 5-(2,6-dideoxy-2-fluoro-α-L-mannopyranosyloxy)-6-hydroxynaphtho[2,3-f]quinoline-7,12-dione represented by the following formula (Ia¹-3)

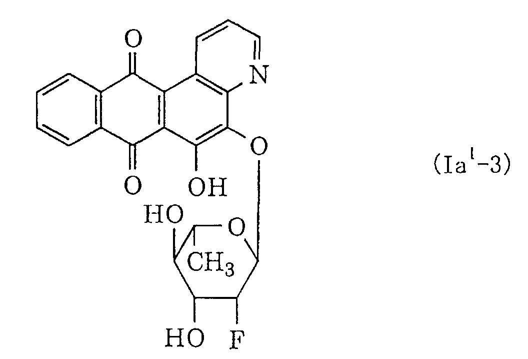

9. A compound as claimed in Claim 6, wherein the compound of general formula (Ia¹) is 5-(2,6-dideoxy-2-fluoro-α-L-galactopyranosyloxy)-6-hydroxynaphtho[2,3-f]quinoline-7,12-dione represented by the following formula (Ia¹-4) 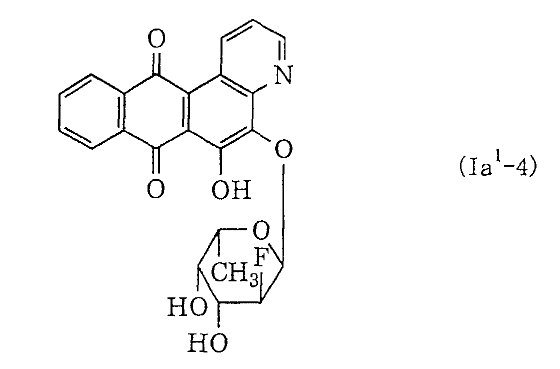 or is 5-(2,6-dideoxy-2-fluoro-β-L-galactopyranosyloxy)-6-hydroxynaphtho[2,3-f]quinoline-7,12-dione represented by the following formula (Ia¹-5)

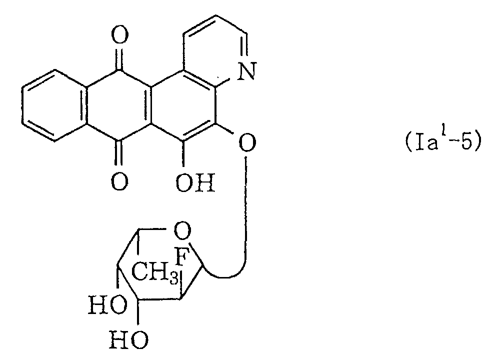

10. A compound as claimed in Claim 3, wherein the compound of general formula (Ia) is a 5-(4-amino-2,4,6-trideoxy-2-substituted-L or D-talopyranosyloxy or galactopyranosyloxy or mannopyranosyloxy)-6-hydroxynaphtho[2,3-f]quinoline-7,12-dione represented by the following general formula (Ia²) 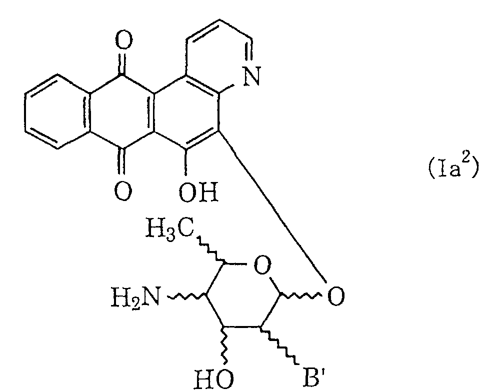 wherein B' is an electron-withdrawing group having the same meaning as defined in Claim 3.

11. A compound as claimed in Claim 10, wherein the compound of general formula (Ia²) is 5-(4-amino-2,4,6-trideoxy-2-fluoro-α-L-mannopyranosyloxy)-6-hydroxynaphtho [2,3-f] quinoline-7, 12-dione of the following formula (Ia²-1) 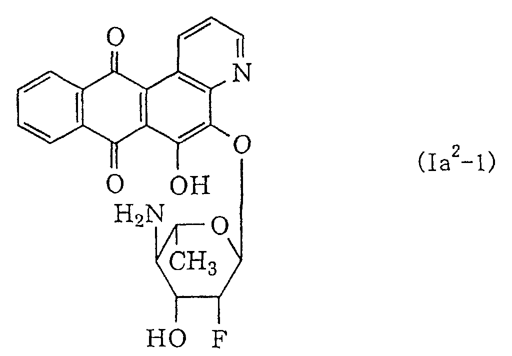 or is 5-(4-amino-2,4,6-trideoxy-2-fluoro-α-L-talopyranosyloxy) -6-hydroxynaphtho[2,3-f] quinoline-7, 12-dione of the following formula (Ia²-2)

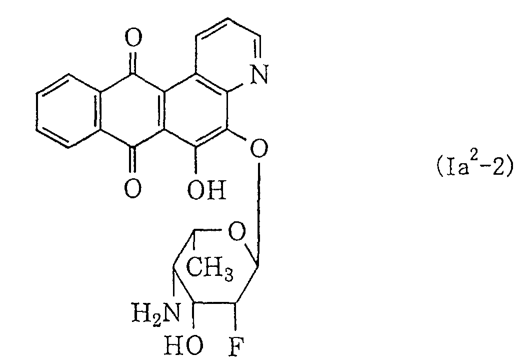

12. A compound as claimed in Claim 3, wherein the compound of general formula (Ia) is a 5-(3-amino-2,3,6-trideoxy-2-substituted-L or D-talopyranosyloxy or galactopyranosyloxy or mannopyranosyloxy)-6-hydroxynaphtho[2,3-f]quinoline-7,12-dione represented by the following general formula (Ia³) 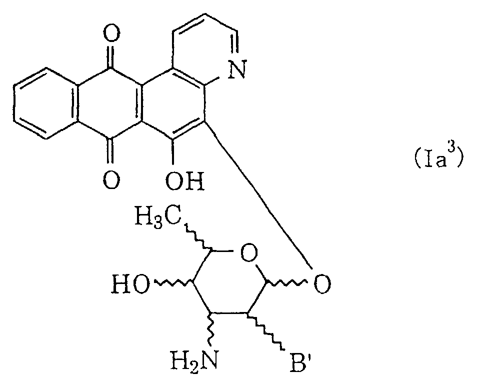 where B' is an electron-withdrawing group as defined in Claim 3.

13. A compound as claimed in Claim 12, wherein the compound of general formula (Ia³) is 5-(3-amino-2,3,6-trideoxy-2-fluoro-α-L-talopyranosyloxy)-6-hydroxynaphtho [2,3-f] quinoline-7,12-dione of the following formula (Ia³-1)

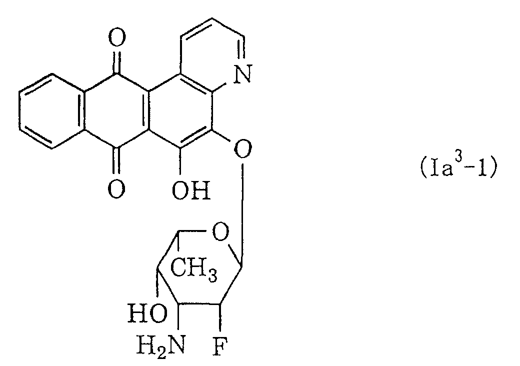

14. A compound as claimed in Claim 3, wherein the compound of general formula (Ia) is a 5-(2,6-dideoxy-2-substituted-3-mono-O-aminoalkanoyl- or 4-mono-O-aminoalkanoyl- or 3,4-di-O-aminoalkanoyl-L or D-talopyranosyloxy or galactopyranosyloxy or mannopyranosyloxy)-6-hydroxynaphtho [2,3-f]quinoline-7,12-dione represented by the following general formula (Ia⁴) 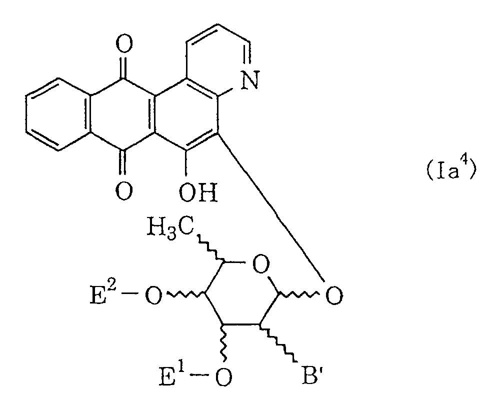 wherein B' is an electron-withdrawing group as defined in Claim 3, and E¹ is hydrogen atom or an α-amino acid residue chosen from glycyl group, alanyl group, valyl group, leucyl group, isoleucyl group and phenylalanyl group, and E² is hydrogen atom or an α-amino acid residue chosen from glycyl group, alanyl group, valyl group, leucyl group, isoleucyl group and phenylalanyl group, provided that E¹ and E² do not represent hydrogen atom, simultaneously.

15. A compound as claimed in Claim 14, wherein the compound of general formula (Ia⁴) is 5-(3-O-L-alanyl-2,6-dideoxy-2-fluoro-α-L-talopyranosyloxy)-6-hydroxynaphtho [2,3-f]quinoline-7,12-dione represented by the following formula (Ia⁴-1) 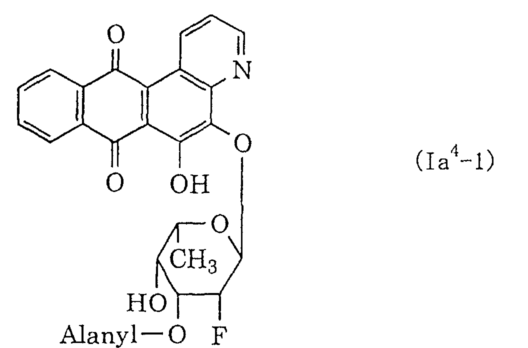 where Alanyl denotes L-alanyl group of formula H₃CCH (-NH₂)-CO-, or is 5-(4-O-L-alanyl-2,6-dideoxy-2-fluoro-α-L-talopyranosyloxy)-6-hydroxynaphtho[2,3-f]quinoline-7,12-dione represented by the following formula (Ia⁴-2) 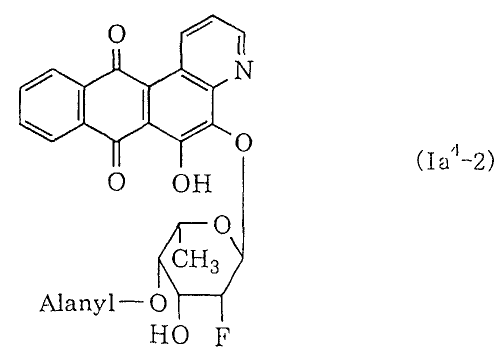 where Alanyl has the same meaning as defined above, or is 5- (3, 4-di-O-L-alanyl-2,6-dideoxy-2-fluoro-α-L-talopyranosyloxy) -6-hydroxynaphtho[2,3-f] quinoline-7,12-dione represented by the following formula (Ia⁴-3) 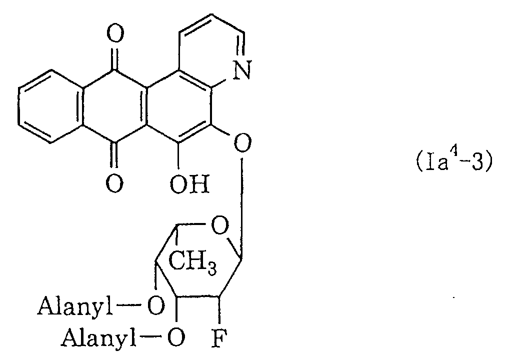 where Alanyl has the same meaning as defined above.

16. A compound as claimed in Claim 3, wherein the compound of general formula (Ia) is a 5-(2,6-dideoxy-2-substituted-3-mono-O-acyl or 4-mono-O-acyl or 3,4-di-O-acyl-L or D-talopyranosyloxy or galactopyranosyloxy or mannopyranosyloxy) -6-hydroxynaphtho[2,3-f]quinoline-7, 12-dione represented by the following general formula (Ia⁵) 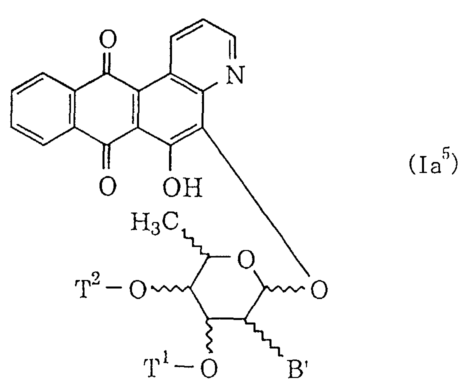 where B' is an electron-withdrawing group as defined in Claim 3, and T¹ is hydrogen atom or an acyl group chosen from formyl group, acetyl group, propionyl group, benzoyl group and benzylcarbonyl group, and T² has the same meaning as defined for T¹, provided that T¹ and T² do not represent hydrogen atom, simultaneously.

17. A compound as claimed in Claim 1, wherein the compound of general formula (I) is a 5-(2, 6-dideoxy-6,6,6-trifluoro-3-O-substituted or unsubstituted- or 4-O-substituted or unsubstituted- or 3,4-O-di-substituted or unsubstituted-glycosyloxy)-6-hydroxynaphtho[2,3-f]-quinoline-7,12-diones represented by the following formula (Ib) 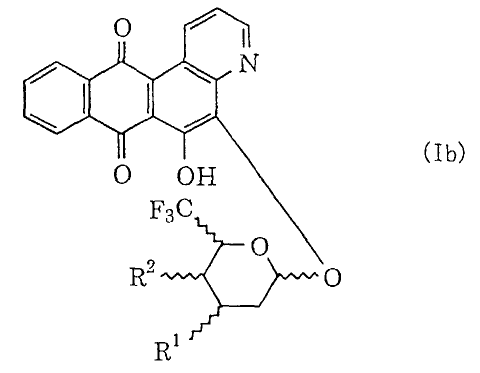 wherein R¹ is hydroxyl group or amino group, or a hydroxyl group having been esterified with an α-amino acid residue of the following formula (a) 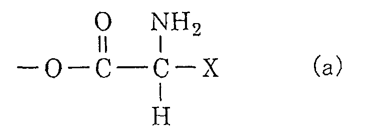 where X is hydrogen atom or such an alkyl group or such a substituted alkyl group which is known to be combined to the α-carbon atom of a known α-amino acid molecule; or R¹ is a hydroxyl group having been esterified with an acyl group of the following formula (b) 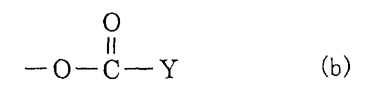 where Y is hydrogen atom or a lower alkyl group, or an aryl group, particularly phenyl group or a substituted phenyl group, or Y is an aralkyl group, particularly benzyl group or a substituted benzyl group; or R¹ is a hydroxyl group having been O-glycosylated with a pentose or a hexose, and R² has the same meaning as defined for R¹.

18. A compound as claimed in Claim 17, wherein the glycosyl group which is present in the compound of general formula (Ib) and which is represented by the following formula (c") 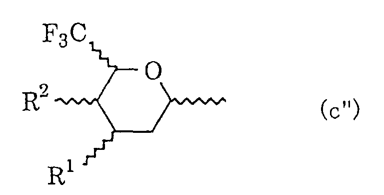 is such a glycosyl group which has the configuration of 2,6-dideoxy-6, 6, 6-trifluoro-α or β-L or D-ribo-hexopyranosyl group; or 2,6-dideoxy-6,6,6-trifluoro-α or β-L or D-arabino-hexopyranosyl group; or 2,6-di-deoxy-6,6,6-trifluoro-α or β-L or D-xylo-hexopyranosyl group; or 2,6-dideoxy-6,6,6-trifluoro-α or β-L or D-lyxo-hexopyranosyl group.

19. A compound as claimed in Claim 17, wherein the compound is such a compound of general formula (Ib) in which R¹ is hydroxyl group, amino group, glycyloxy group, alanyloxy group or acetoxy group, and R² is hydroxyl group, amino group, glycyloxy group, alanyloxy group or acetoxy group.

20. A compound as claimed in Claim 17, wherein the compound of general formula (Ib) is a 5-(2,6-dideoxy-6,6,6-trifluoro-4-substituted or unsubstituted-α or β-L or D-ribo-hexopyranosyloxy or 2,6-dideoxy-6,6,6-trifluoro-4-substituted or unsubstituted-α or β-L or D-arabino-hexopyranosyloxy or 2,6-dideoxy-6,6,6-trifluoro-4-substituted or unsubstituted -α or β-L or D-xylo-hexopyranosyloxy or 2,6-dideoxy-6,6,6-trifluoro-4-substituted or unsubstituted-α or β-L or D-lyxo-hexopyranosyloxy)-6-hydroxynaphtho[2,3-f]quinoline-7,12-dione represented by the following general formula (Ib¹) 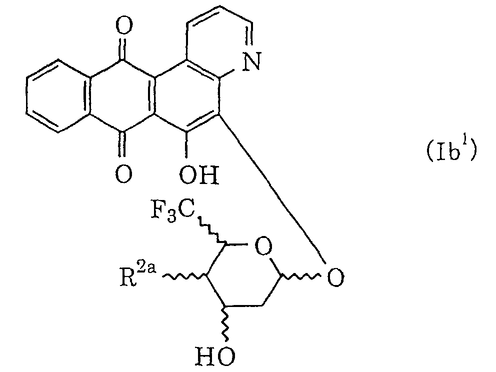 wherein R^{2a} is hydroxyl group, amino group, glycyloxy group, alanyloxy group or acetoxy group.

21. A compound as claimed in Claim 20, wherein the compound of general formula (Ib¹) is 5-(2,6-dideoxy-6,6,6-trifluoro-α-L-lyxo-hexopyranosyloxy)-6-hydroxynaphtho [2,3-f]quinoline-7,12-dione represented by the following formula (Ib¹-1) 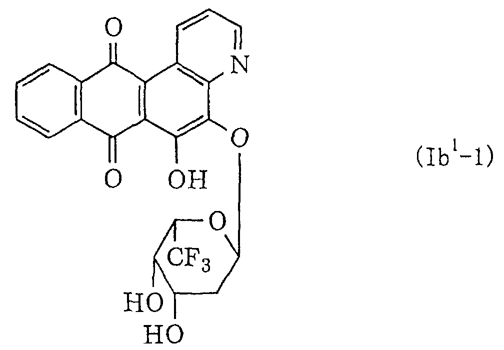 or is 5-(2,6-dideoxy-6,6,6-trifluoro-β-L-lyxo-hexopyranosyloxy) -6-hydroxynaphtho[2,3-f]quinoline-7,12-dione represented by the following formula (Ib¹-2)

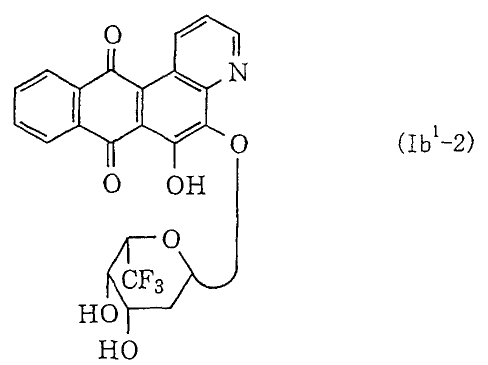

22. A 5-L or D-glycosyloxy-6-hydroxy or 6-O-substituted hydroxynaphtho[2,3-f]quinoline-7,12-dione represented by the following general formula (V) 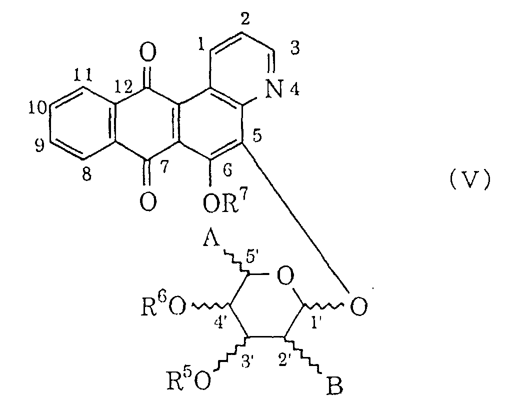 wherein A is methyl group or trifluoromethyl group, and B is hydrogen atom, or an electron-withdrawing group chosen from fluoro group, chloro group, bromo group, iodo group, difluoro group, an alkoxy group of 1-5 carbon atoms and cyano group, provided that B stands for hydrogen atom when A is trifluoromethyl group and that B stands for an electron-withdrawing group when A is methyl group, and R⁵ is hydrogen atom or an ω-amino acid residue of the following formula (d)
-CO-G-NH₂ (d)
where G is a linear alkylene group of 2-6 carbon atoms which may further be substituted by a (C₁-C₆) alkyl group, and R⁶ and R⁷ have the same meanings as defined for R⁵, provided that all of R⁵, R⁶ and R⁷ simultaneously do not represent hydrogen atom, and a pharmaceutically acceptable salt thereof.

23. A compound as claimed in Claim 22, wherein the compound of general formula (V) is a 5-(2,6-dideoxy-2-fluoro -3-mono-O-aminoalkanoyl- or 4-mono-O-aminoalkanoyl- or 3,4-di-O-aminoalkanoyl-L or D-talopyranosyloxy or galactopyranosyloxy or mannopyranosyloxy)-6-hydroxy or 6-aminoalkanoyloxynaphtho [2,3-f]quinoline-7,12-dione represented by the following general formula (Va) 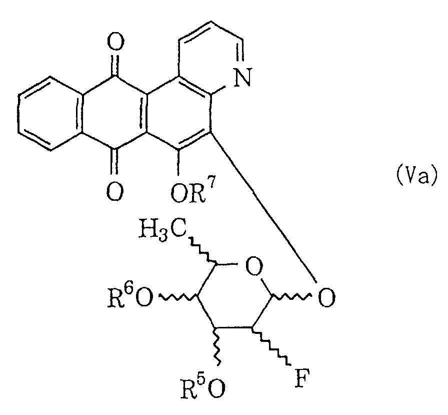 where R⁵, R⁶ and R⁷ each are an ω-amino acid residue of formula (d) having the same meaning as defined in Claim 22, or a hydrogen atom, and said ω-amino acid residue is preferably 3-aminopropionyl group, 4-aminobutylyl group, 5-aminopentanoyl group, 6-aminohexanoyl group, 7-amino-heptanoyl group, 4-amino-2-methylbutylyl group or 5-amino-3-ethylpentanoyl group, provided that all of R⁵, R⁶ and R⁷ do not represent hydrogen atom, simultaneously.

24. A compound as claimed in Claim 23, wherein the compound of general formula (Va) is a 5-(2,6-dideoxy-2-fluoro-3-mono-O-aminoalkanoyl- or 4-mono-O-aminoalkanoyl-or 3,4-di-O-aminoalkanoyl-L or D-talopyranosyloxy)-6-hydroxynaphtho[2,3-f]quinoline-7,12-dione represented by the following general formula (Va-1) 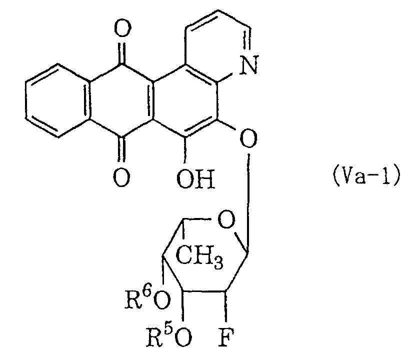 where R⁵ is hydrogen atom, or an ω-amino acid residue of formula (d) defined in Claim 22, and R⁶ has the same meaning as defined for R⁵, provided that R⁵ andR⁶ do not represent hydrogen atom, simultaneously.

25. A compound as claimed in Claim 23, wherein the compound of general formula (Va) is a 5-(2,6-dideoxy-2-fluoro-3-mono-O-aminoalkanoyl- or 4-mono-O-aminoalkanoyl-or 3,4-di-O-aminoalkanoyl-L or D-galactopyranosyloxy)-6-hydroxy or 6-aminoalkanoyloxy-naphtho[2,3-f]-quinoline-7,12-dione represented by the following general formula (Va-2) 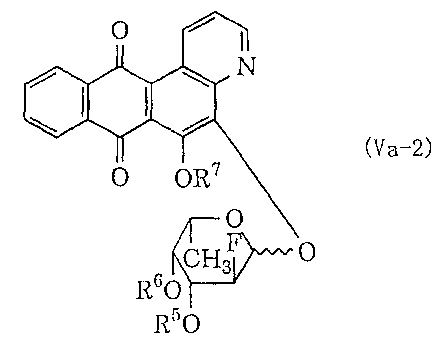 wherein R⁵ is hydrogen atom, or an ω-amino acid residue of formula (d) defined in Claim 22, and R⁶ and R⁷ each have the same meanings as defined for R⁵, provided that R⁵, R⁶ and R⁷ do not represent hydrogen atom, simultaneously.

26. A compound as claimed in Claim 23, wherein the compounds of general formula (Va) is a 5-(2,6-dideoxy-2-fluoro-3-mono-O-aminoalkanoyl- or 4-mono-O-aminoalkanoyl-or 3,4-di-O-aminoalkanoyl-L or D-mannopyranosyloxy)-6-hydroxynaphtho[2,3-f]quinoline-7,12-dione represented by the following general formula (Va-3) 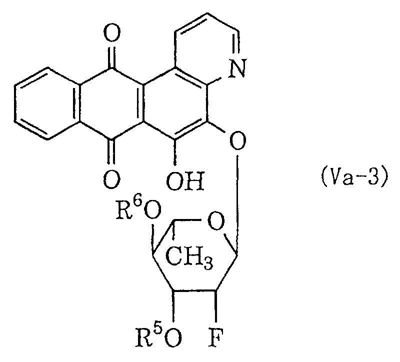 where R⁵ is hydrogen atom or an ω-amino acid residue of formula (d) as defined in Claim 22, and R⁶ has the same meaning as defined for R⁵, provided that R⁵ and R⁶ do not represent hydrogen atom, simultaneously.

27. A compound as claimed in Claim 24, wherein the compound of general formula (Va-1) is 5-[3-O-(3-aminopropionyl)-2,6-dideoxy-2-fluoro-α-L-talopyranosyloxy]-6-hydroxynaphtho [2,3-f]quinoline-7,12-dione (Compound N of this invention); 5-[4-O-(3-aminopropionyl)-2,6-dideoxy-2-fluoro-α-L-talopyranosyloxy]-6-hydroxynaphtho[2,3-f]-quinoline-7,12-dione (Compound O of this invention); or 5-[3,4-di-O-(3-aminopropionyl)-2,6-dideoxy-2-fluoro-α-L-talopyranosyloxy] -6-hydroxynaphtho[2,3-f]quinoline-7,12-dione (Compound P of this invention).

28. A compound as claimed in Claim 25, wherein the compound of general formula (Va-2) is 5-[3,4-di-O-(3-aminopropionyl)-2,6-dideoxy-2-fluoro-α-L-galactopyranosyloxy] -6-hydroxynaphtho[2,3-f]quinoline-7,12-dione(Compound Q of this invention); 5-[3-O-(3-aminopropionyl)-2,6-dideoxy-2-fluoro-β-L-galactopyranosyloxy]-6-hydroxynaphtho[2,3-f]-quinoline-7,12-dione (Compound R of this invention); 5-[3,4-di-O-(3-aminopropionyl)-2,6-dideoxy-2-fluoro-β-L-galactopyranosyloxy]-6-hydroxynaphtho[2,3-f]quinoline-7,12-dione(Compound S of this invention); or 5-[3,4-di-O-(3-aminopropionyl)-2,6-dideoxy-2-fluoro-β-L-galactopyranosyloxy] -6-(3-aminopropionyloxy)-naphtho[2,3-f]quinoline-7,12-dione (Compound T of this invention).

29. An anticancer or antitumor composition comprising as an active ingredient a 5-glycosyloxy-6-hydroxynaphtho[2,3-f] quinoline-7,12-dione of general formula (I) as defined in Claim 1 or a pharmaceutically acceptable salt thereof, in combination with a pharmaceutically acceptable solid or liquid carrier or carriers.

30. An anticancer or antitumor composition comprising as an active ingredient a 5-glycosyloxy-6-hydroxynaphtho[2,3-f] quinoline-7, 12-dione of general formula (Ia) as defined in Claim 3 or a pharmaceutically acceptable salt thereof, in combination with a pharmaceutically acceptable solid or liquid carrier or carriers.

31. An anticancer or antitumor composition comprising as an active ingredient a 5-glycosyloxy-6-hydroxynaphtho [2,3-f]quinoline-7, 12-dione of general formula (Ib) as defined in Claim 17 or a pharmaceutically acceptable salt thereof, in combination with a pharmaceutically acceptable solid or liquid carrier or carriers.

32. An anticancer or antitumor composition comprising as an active ingredient a 5-L or D-glycosyloxy-6-hydroxy or 6-O-substituted hydroxynaphtho[2,3-f]quinoline-7,12-dione of general formula (V) as defined in Claim 22 or a pharmaceutically acceptable salt thereof, in combination with a pharmaceutically acceptable solid or liquid carrier or carriers.

33. A composition as claimed in Claim 32, wherein the active ingredient is a compound of general formula (Va-1) as defined in Claim 24, or a compound of general formula (Va-2) as defined in Claim 25, or a compound of general formula (Va-3) as defined in Claim 26, or a pharmaceutically acceptable salt thereof.
